# EUROPEAN PATENT APPLICATION

(11) **EP 2 386 545 A1**
(43) Date of publication of application: **16.11.2011**
(21) Application number: 11170364.1
(22) Date of filing: 17.04.2002
(51) Int. Cl.: C07D 213/75, C07D 217/22, C07D 401/12, C07D 215/38, C07D 403/12, A61K 31/44, A61K 31/47, A61P 29/00

(54) **Heteroaryl ureas containing nitrogen hetero-atoms as P38 kinase inhibitors**

(30) Priority: 20.04.2001 US 838286
(62) Divisional of application: 02725709.6
(71) Applicant: Bayer Healthcare LLC, Tarrytown, NY 10591-5097 (US)
(72) Inventor: Dumas, Jacques, Bethany, CT Connecticut 06524 (US); Riedl, Bernd, 42329 Wuppertal (DE); Khire, Uday, Hamden, CT Connecticut 06518 (US); Sibley, Robert, N., North Haven, CT Connecticut 06473 (US); Hatoum-Mokdad, Holia, Hamden, CT Connecticut 06514 (US); Monahan, Mary-Katherine, Hamden, CT Connecticut 06517 (US); Gunn, David, E., Hamden, CT Connecticut 06517 (US); Lowinger, Timothy, B., 42117 Wuppertal (DE); Scott, William, Guilford, CT Connecticut 06437 (US); Smith, Roger, A., Madison, CT Connecticut 06443 (US); Wood, Jill, E., North Haven, CT Connecticut 06473 (US)
(74) Representative: Hart-Davis, Jason

(57) **Abstract**

This invention relates to the use of a group of heteroaryl ureas containing nitrogen in treating p38 mediated diseases, and pharmaceutical compositions for use in such therapy.

## Description

### Cross Reference to Related Applications

This is a continuation-in-part of application Serial No. 09/778,039 filed February 7, 2001, which is a continuation-in-part of Serial No. 09/425,229 filed October 22, 1999, which is a continuation of 09/257,265 filed February 25, 1999 which claims priority to provisional application 60/115,878, filed January 13, 1999.

### Field of the Invention

This invention relates to the use of a group of heteroaryl ureas containing nitrogen hetero atoms in treating cytokine mediated diseases and proteolytic enzyme mediated diseases, and pharmaceutical compositions for use in such therapy.

### Background of the Invention

Two classes of effector molecules which are critical for the progression of rheumatoid arthritis are pro-inflammatory cytokines and tissue degrading proteases. Recently, a family of kinases was described which is instrumental in controlling the transcription and translation of the structural genes coding for these effector molecules.

The mitogen-activated protein (MAP) kinase family is made up of a series of structurally related proline-directed serine/threonine kinases which are activated either by growth factors (such as EGF) and phorbol esters (ERK), or by IL-1, TNFα or stress (p38, JNK). The MAP kinases are responsible for the activation of a wide variety of transcription factors and proteins involved in transcriptional control of cytokine production. A pair of novel protein kinases involved in the regulation of cytokine synthesis was recently described by a group from SmithKline Beecham (Lee et al. Nature 1994, 372, 739). These enzymes were isolated based on their affinity to bond to a class of compounds, named CSAIDSs (cytokine suppressive anti-inflammatory drugs) by SKB. The CSAIDs, bicyclic pyridinyl imidazoles, have been shown to have cytokine inhibitory activity both *in vitro* and *in vivo.* The isolated enzymes, CSBP-1 and -2 (CSAID binding protein 1 and 2) have been cloned and expressed. A murine homologue for CSBP-2, p38, has also been reported (Han et al. Science 1994, 265, 808).

Early studies suggested that CSAIDs function by interfering with m-RNA translational events during cytokine biosynthesis. Inhibition of p38 has been shown to inhibit both cytokine production (eg., TNFα, IL-1, IL-6, IL-8) and proteolytic enzyme production (eg., MMP-1, MMP-3) *in vitro* and/or *in vivo.*

Clinical studies have linked TNFα production and/or signaling to a number of diseases including rheumatoid arthritis (Maini. J. Royal Coll. Physicians London 1996, 30, 344). In addition, excessive levels of TNFα have been implicated in a wide variety of inflammatory and/or immunomodulatory diseases, including acute rheumatic fever (Yegin et al. Lancet 1997, 349, 170), bone resorption (Pacifici et al. J. Clin. Endocrinol. Metabol. 1997, 82, 29), postmenopausal osteoperosis (Pacifici et al. J. Bone Mineral Res. 1996, 11, 1043), sepsis (Blackwell et al. Br. J. Anaesth. 1996, 77, 110), gram negative sepsis (Debets et al. Prog. Clin. Biol. Res. 1989, 308, 463), septic shock (Tracey et al. Nature 1987, 330, 662; Girardin et al. New England J. Med. 1988, 319, 397), endotoxic shock (Beutler et al. Science 1985, 229, 869; Ashkenasi et al. Proc. Nat'l. Acad. Sci. USA 1991, 88, 10535), toxic shock syndrome, (Saha et al. J. Immunol. 1996, 157, 3869; Lina et al. FEMS Immunol. Med. Microbiol. 1996, 13, 81), systemic inflammatory response syndrome (Anon. Crit. Care Med. 1992, 20, 864), inflammatory bowel diseases (Stokkers et al. J. Inflamm. 1995-6, 47, 97) including Crohn's disease (van Deventer et al. Aliment. Pharmacol. Therapeu. 1996, 10 (Suppl. 2), 107; van Dullemen et al. Gastroenterology 1995, 109, 129) and ulcerative colitis (Masuda et al. J. Clin. Lab. Immunol. 1995, 46, 111), Jarisch-Herxheimer reactions (Fekade et al. New England J. Med. 1996, 335, 311), asthma (Amrani et al. Rev. Malad. Respir. 1996, 13, 539), adult respiratory distress syndrome (Roten et al. Am. Rev. Respir. Dis. 1991, 143, 590; Suter et al. Am. Rev. Respir. Dis. 1992, 145, 1016), acute pulmonary fibrotic diseases (Pan et al. Pathod. Int. 1996, 46, 91), pulmonary sarcoidosis (Ishioka et al. Sarcoidosis Vasculitis Diffuse Lung Dis. 1996, 13, 139), allergic respiratory diseases (Casale et al. Am. J. Respir. Cell Mol. Biol. 1996, 15, 35), silicosis (Gossart et al. J. Immunol. 1996, 156, 1540; Vanhee et al. Eur. Respir. J. 1995, 8, 834), coal worker's pneumoconiosis (Borm et al. Am. Rev. Respir. Dis. 1988, 138, 1589), alveolar injury (Horinouchi et al. Am. J. Respir. Cell Mol. Biol. 1996, 14, 1044), hepatic failure (Gantner et al. J. Pharmacol. Exp. Therap. 1997, 280, 53), liver disease during acute inflammation (Kim et al. J. Biol. Chem. 1997, 272, 1402), severe alcoholic hepatitis (Bird et al. Ann. Intern. Med. 1990, 112, 917), malaria (Grau et al. Immunol. Rev. 1989, 112, 49; Taverne et al. Parasitol. Today 1996, 12, 290) including Plasmodium falciparum malaria (Perlmann et al. Infect. Immunit. 1997, 65, 116) and cerebral malaria (Rudin et al. Am. J. Pathol. 1997, 150, 257), non-insulin-dependent diabetes mellitus (NIDDM; Stephens et al. J. Biol. Chem. 1997, 272, 971; Ofei et al. Diabetes 1996, 45, 881), congestive heart failure (Doyama et al. Int. J. Cardiol. 1996, 54, 217; McMurray et al. Br. Heart J. 1991, 66, 356), damage following heart disease (Malkiel et al. Mol. Med. Today 1996, 2, 336), atherosclerosis (Parums et al. J. Pathod. 1996, 179, A46), Alzheimer's disease (Fagarasan et al. Brain Res. 1996, 723, 231; Aisen et al. Gerontology 1997, 43, 143), acute encephalitis (Ichiyama et al. J. Neurol. 1996, 243, 457), brain injury (Cannon et al. Crit. Care Med. 1992, 20, 1414; Hansbrough et al. Surg. Clin. N. Am. 1987, 67, 69; Marano et al. Surg. Gynecol. Obstetr. 1990, 170, 32), multiple sclerosis (M.S.; Coyle. Adv. Neuroimmunol. 1996, 6, 143; Matusevicius et al. J. Neuroimmunol. 1996, 66, 115) including demyelation and oligiodendrocyte loss in multiple sclerosis (Brosnan et al. Brain Pathol. 1996, 6, 243), advanced cancer (MucWierzgon et al. J. Biol. Regulators Homeostatic Agents 1996, 10, 25), lymphoid malignancies (Levy et al. Crit. Rev. Immunol. 1996, 16, 31), pancreatitis (Exley et al. Gut 1992, 33, 1126) including systemic complications in acute pancreatitis (McKay et al. Br. J. Surg. 1996, 83, 919), impaired wound healing in infection inflammation and cancer (Buck et al. Am. J. Pathol. 1996, 149, 195), myelodysplastic syndromes (Raza et al. Int. J. Hematol. 1996, 63, 265), systemic lupus erythematosus (Maury et al. Arthritis Rheum. 1989, 32, 146), biliary cirrhosis (Miller et al. Am. J. Gasteroenterolog. 1992, 87, 465), bowel necrosis (Sun et al. J. Clin. Invest. 1988, 81, 1328), psoriasis (Christophers. Austr. J. Dermatol. 1996, 37, S4), radiation injury (Redlich et al. J. Immunol. 1996, 157, 1705), and toxicity following administration of monoclonal antibodies such as OKT3 (Brod et al. Neurology 1996, 46, 1633). TNFα levels have also been related to host-versus-graft reactions (Piguet et al. Immunol. Ser. 1992, 56, 409) including ischemia reperfusion injury (Colletti et al. J. Clin. Invest. 1989, 85, 1333) and allograft rejections including those of the kidney (Maury et al. J. Exp. Med. 1987, 166, 1132), liver (Imagawa et al. Transplantation 1990, 50, 219), heart (Bolling et al. Transplantation 1992, 53, 283), and skin (Stevens et al. Transplant. Proc. 1990, 22, 1924), lung allograft rejection (Grossman et al. Immunol. Allergy Clin. N. Am. 1989, 9, 153) including chronic lung allograft rejection (obliterative bronchitis; LoCicero et al. J. Thorac. Cardiovasc. Surg. 1990, 99, 1059), as well as complications due to total hip replacement (Cirino et al. Life Sci. 1996, 59, 86). TNFα has also been linked to infectious diseases (review: Beutler et al. Crit. Care Med. 1993, 21, 5423; Degre. Biotherapy 1996, 8, 219) including tuberculosis (Rook et al. Med. Malad. Infect. 1996, 26, 904), Helicobacter pylori infection during peptic ulcer disease (Beales et al. Gastroenterology 1997, 112, 136), Chaga's disease resulting from Trypanosoma cruzi infection (Chandrasekar et al. Biochem. Biophys. Res. Commun. 1996, 223, 365), effects of Shiga-like toxin resulting from E. coli infection (Harel et al. J. Clin. Invest. 1992, 56, 40), the effects of enterotoxin A resulting from Staphylococcus infection (Fischer et al. J. Immunol. 1990, 144, 4663), meningococcal infection (Waage et al. Lancet 1987, 355; Ossege et al. J. Neurolog. Sci. 1996, 144, 1), and infections from Borrelia burgdorferi (Brandt et al. Infect. Immunol. 1990, 58, 983), Treponema pallidum (Chamberlin et al. Infect. Immunol. 1989, 57, 2872), cytomegalovirus (CMV; Geist et al. Am. J. Respir. Cell Mol. Biol. 1997, 16, 31), influenza virus (Beutler et al. Clin. Res. 1986, 34, 491a), Sendai virus (Goldfield et al. Proc. Nat'l. Acad. Sci. USA 1989, 87, 1490), Theiler's encephalomyelitis virus (Sierra et al. Immunology 1993, 78, 399), and the human immunodeficiency virus (HIV; Poli. Proc. Nat'l. Acad. Sci. USA 1990, 87, 782; Vyakaram et al. AIDS 1990, 4, 21; Badley et al. J. Exp. Med. 1997, 185, 55).

Because inhibition of p38 leads to inhibition of TNFα production, p38 inhibitors will be useful in treatment of the above listed diseases.

A number of diseases are thought to be mediated by excess or undesired matrix-destroying metalloprotease (MMP) activity or by an imbalance in the ratio of the MMPs to the tissue inhibitors of metalloproteinases (TIMPs). These include osteoarthritis (Woessner et al. J. Biol. Chem. 1984, 259, 3633), rheumatoid arthritis (Mullins et al. Biochim. Biophys. Acta 1983, 695, 117; Woolley et al. Arthritis Rheum. 1977, 20, 1231; Gravallese et al. Arthritis Rheum. 1991, 34, 1076), septic arthritis (Williams et al. Arthritis Rheum. 1990, 33, 533), tumor metastasis (Reich et al. Cancer Res. 1988, 48, 3307; Matrisian et al. Proc. Nat'l. Acad. Sci., USA 1986, 83, 9413), periodontal diseases (Overall et al. J. Periodontal Res. 1987, 22, 81), corneal ulceration (Burns et al. Invest. Opthalmol. Vis. Sci. 1989, 30, 1569), proteinuria (Baricos et al. Biochem. J. 1988, 254, 609), coronary thrombosis from atherosclerotic plaque rupture (Henney et al. Proc. Nat'l. Acad. Sci., USA 1991, 88, 8154), aneurysmal aortic disease (Vine et al. Clin. Sci. 1991, 81, 233), birth control (Woessner et al. Steroids 1989, 54, 491), dystrophobic epidermolysis bullosa (Kronberger et al. J. Invest. Dermatol. 1982, 79, 208), degenerative cartilage loss following traumatic joint injury, osteopenias mediated by MMP activity, tempero mandibular joint disease, and demyelating diseases of the nervous system (Chantry et al. J. Neurochem. 1988, 50, 688).

Because inhibition of p38 leads to inhibition of MMP production, p38 inhibitors will be useful in treatment of the above listed diseases.

Inhibitors of p38 are active in animal models of TNFα production, including a muirne lipopolysaccharide (LPS) model of TNFα production. Inhibitors of p38 are active in a number of standard animal models of inflammatory diseases, including carrageenan-induced edema in the rat paw, arachadonic acid-induced edema in the rat paw, arachadonic acid-induced peritonitis in the mouse, fetal rat long bone resorption, murine type II collagen-induced arthritis, and Fruend's adjuvant-induced arthritis in the rat. Thus, inhibitors of p38 will be useful in treating diseases mediated by one or more of the above-mentioned cytokines and/or proteolytic enzymes.

The need for new therapies is especially important in the case of arthritic diseases. The primary disabling effect of osteoarthritis, rheumatoid arthritis and septic arthritis is the progressive loss of articular cartilage and thereby normal joint function. No marketed pharmaceutical agent is able to prevent or slow this cartilage loss, although nonsteroidal antiinflammatory drugs (NSAIDs) have been given to control pain and swelling. The end result of these diseases is total loss of joint function which is only treatable by joint replacement surgery. P38 inhibitors will halt or reverse the progression of cartilage loss and obviate or delay surgical intervention.

Several patents have appeared claiming polyarylimidazoles and/or compounds containing polyarylimidazoles as inhibitors of p38 (for example, Lee et al. WO 95/07922; Adams et al. WO 95/02591; Adams et al. WO 95/13067; Adams et al. WO 95/31451). It has been reported that arylimidazoles complex to the ferric form of cytochrome P450_{cam} (Harris et al. Mol. Eng. 1995, 5, 143, and references therein), causing concern that these compounds may display structure-related toxicity (Howard-Martin et al. Toxicol. Pathol. 1987, 15, 369). Therefore, there remains a need for improved p38 inhibitors.

### Summary of the Invention

This invention provides compounds, generally described as heteroaryl ureas containing nitrogen hetero atoms, including pyridine, quinoline and isoquinoline ureas, which inhibit p38 mediated events and thus inhibit the production of cytokines (such as TNFα, IL-1 and IL-8) and proteolytic enzymes (such as MMP-1 and MMP-3). The invention also provides compositions which contain heteroaryl ureas and a method of treating a cytokine mediated disease state in humans or mammals with heteroaryl ureas, wherein the cytokine is one whose production is affected by p38. Examples of such cytokines include, but are not limited to TNFα, IL-1 and IL-8. The invention also provides a method of treating a protease mediated disease state in humans or mammals, wherein the protease is one whose production is affected by p38, e.g. disease states mediated by one or more cytokines or proteolytic enzymes produced and/or activated by a p38 mediated process. Examples of such proteases include, but are not limited to collagenase (MMP-1) and stromelysin (MMP-3).

Accordingly, these compounds are useful therapeutic agents for such acute and chronic inflammatory and/or immunomodulatory diseases as rheumatoid arthritis, osteoarthritis, septic arthritis, rheumatic fever, bone resorption, postmenopausal osteoperosis, sepsis, gram negative sepsis, septic shock, endotoxic shock, toxic shock syndrome, systemic inflammatory response syndrome, inflammatory bowel diseases including Crohn's disease and ulcerative colitis, Jarisch-Herxheimer reactions, asthma, adult respiratory distress syndrome, acute pulmonary fibrotic diseases, pulmonary sarcoidosis, allergic respiratory diseases, silicosis, coal worker's pneumoconiosis, alveolar injury, hepatic failure, liver disease during acute inflammation, severe alcoholic hepatitis, malaria including Plasmodium falciparum malaria and cerebral malaria, non-insulin-dependent diabetes mellitus (NIDDM), congestive heart failure, damage following heart disease, atherosclerosis, Alzheimer's disease, acute encephalitis, brain injury, multiple sclerosis including demyelation and oligiodendrocyte loss in multiple sclerosis, advanced cancer, lymphoid malignancies, tumor metastasis, pancreatitis, including systemic complications in acute pancreatitis, impaired wound healing in infection, inflammation and cancer, periodontal diseases, corneal ulceration, proteinuria, myelodysplastic syndromes, systemic lupus erythematosus, biliary cirrhosis, bowel necrosis, psoriasis, radiation injury, toxicity following administration of monoclonal antibodies such as OKT3, host-versus-graft reactions including ischemia reperfusion injury and allograft rejections including kidney, liver, heart, and skin allograft rejections, lung allograft rejection including chronic lung allograft rejection (obliterative bronchitis) as well as complications due to total hip replacement, and infectious diseases including tuberculosis, Helicobacter pylori infection during peptic ulcer disease, Chaga's disease resulting from Trypanosoma cruzi infection, effects of Shiga-like toxin resulting from E. coli infection, effects of enterotoxin A resulting from Staphylococcus infection, meningococcal infection, and infections from Borrelia burgdorferi, Treponema pallidum, cytomegalovirus, influenza virus, Theiler's encephalomyelitis virus, and the human immunodeficiency virus (HIV).

The present invention, therefore, provides hetaryl urea compounds containing nitrogen hetero-atoms, and compositions which comprise hetaryl urea compounds containing nitrogen heteroatoms and a method for treating of p38-mediated disease states in humans or mammals, e.g., disease states mediated by one or more cytokines or proteolytic enzymes produced and/or activated by a p38 mediated process. In these methods a compound of formula I, or a pharmaceutically acceptable salt thereof, is administered,

A-D-B (I).

In formula I,
D is -NH-C(O)-NH-,
A is a substituted or unsubstituted pyridyl, quinolinyl or isoquinoliyl group,
B is a substituted or unsubstituted, up to tricyclic aryl or heteroaryl moiety of up to 50 carbon atoms with a cyclic structure bound directly to D containing at least 5 members with 0-4 members of the group consisting of nitrogen, oxygen and sulfur.

The moiety B is preferably either a substituted or unsubstituted bridged cyclic structure of up to 30 carbon atoms of the formula -L-(ML¹)_{q}, a substituted or unsubstituted 6 member cyclic aryl moiety or hetaryl moiety or a substituted or unsubstituted 2-3 fused ring structure (aryl, hetaryl or both). For Example, B can be phenyl, substituted phenyl, napthyl substituted napthyl, pyridinyl, substituted pyridinyl, pyrimidinyl, substituted pyrimidinyl, quinolinyl, substituted quinolinyl, isoquinolinyl, substituted isoquinolinyl or of the formula -L(ML¹)_{q}.

L in the formula -L(ML¹)_{q} is a 5 or 6 membered cyclic structure bond directly to D, L¹ is a cyclic moiety of at least 5 members,

M is a bridging group having at least one atom and q is an integer of 1-3.

Each cyclic structure of L and L¹ contains from 0-4 members of the group consisting of N, O and S.

The substituents for the groups of A are preferably selected from the group consisting of halogen, up to per-halo, and Wn, where n is 0-3 and each W is independently selected from the group consisting of C₁₋₁₀ alkyl, C₁₋₁₀ alkoxy, C₃₋₁₀ cycloalkyl having at least five cyclic members and 0-3 heteroatoms, C₂₋₁₀ alkenyl, C₁₋₁₀ alkenoyl, substituted C₁₋₁₀ alkyl, substituted C₁₋₁₀ alkoxy, a substituted C₃₋₁₀ cycloalkyl having at least 5 cyclic members and 0-3 heteroatoms selected from N, S and O; substituted C₂₋₁₀ alkenyl, substituted C₁₋₁₀ alkenoyl, C₆-C₁₄ aryl, C₇-C₂₄ alkaryl, C₇-C₂₄ aralkyl, C₃-C₁₂ hetaryl having at least 5 cyclic members and 1-3 heteroatoms selected from O, N and S, C₄-C₂₃ alkheteroaryl having at least 5 cyclic members and 1-3 heteroatoms selected from O, N and S, substituted C₆-C₁₄ aryl, substituted C₃-C₁₂ hetaryl having at least 5 members and 1-3 heteroatoms selected from O, N and S, substituted C₇-C₂₄ aralkyl, substituted C₇-C₂₄ alkaryl, substituted C₄-C₂₃ alkheteroaryl having at least 5 cyclic members and 1-3 heteroatoms selected from O, N and S; -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, with each R⁷ and R^{7'} independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstituted C₂₋₁₀ alkenyl and up to per halosubstituted C₁₋₁₀ alkenoyl, C₃-C₁₀ cycloalkyl having at least 5 cyclic members and 0-3 heteroatoms selected from O, S and N, C₆-C₁₄ aryl, C₃-C₁₀ hetaryl having at least 5 cyclic members and 0-3 heteroatoms selected from O, S and N, up to per halosubstituted C₃-C₁₀ cycloalkyl having at least 6 cyclic members and 0-3 heteroatoms selected from O, S and N, up to per halo substituted C₆-C₁₄ aryl, and up to per halo substituted C₃-C₁₀ hetaryl having at least 6 cyclic members and 0-3 heteroatoms selected from O, S and N.

Where W is a substituted group, it is substituted by halogen, up to per halo, or by one or more substituents independently selected from the group consiting of -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'} with each R⁷ and R^{7'} independently as defined above.

Where B, is substituted, the substituents are selected from the group consisting of halogen, up to per-halo, and Jₙ, where n is 0-3 and each J is independently selected from the group consisting of -CN, -CO₂R⁷, -C(O)NR⁷R^{7'}, -C(O)-R⁷, -NO₂, -OR⁷, -SR⁷, -NR⁷R^{7'}, -NR⁷C(O)OR^{7'}, -NR⁷C(O)R^{7'}, with each R⁷ and R^{7'} independently as defined above , C₁₋₁₀ alkyl, C₁₋₁₀ alkoxy, C₃₋₁₀ cycloalkyl having at least five cyclic members and 0-3 heteroatoms, C₂₋₁₀ alkenyl, C₁₋₁₀ alkenoyl, C₆₋₁₄ aryl, C₃₋₁₂ hetaryl having at least five cyclic members and 1-3 heteroatoms selected from N, S and O, C₇₋₂₄ aralkyl, C₇₋₂₄ alkaryl, C₄-C₂₃ alkheteroaryl having at least 5 cyclic members and 1-3 heteroatoms selected from O, N and S, substituted C₁₋₁₀ alkyl, substituted C₁₋₁₀ alkoxy, substituted C₃₋₁₀ cycloalkyl having at least five cyclic members and 0-3 heteroatoms selected from N, S and O, substituted C₂₋₁₀ alkenyl substituted C₁₋₁₀ alkenoyl, substituted C₆-C₁₂ aryl, substituted C₃₋₁₂ hetaryl having at least five cyclic members and 1-3 heteroatoms selected from N, S and O, substituted C₇₋₂₄ alkaryl , substituted C₇-C₂₄ aralkyl substituted C₄-C₂₃ alkheteroaryl having at least 5 cyclic members and 1-3 heteroatoms selected from O, N and S, and -Q-Ar.

Where J is a substituted group, it is substituted by halogen, up to per halo, or by one or more substituents independently selected from the group consisting of -CN, -CO₂R⁷, -C(O)-R⁷, -C(O)NR⁷R^{7'}, -OR⁷, -SR⁷, -NR⁷R^{7'}, -NO₂, -NR⁷C(O)R^{7'}, and -NR⁷C(O)OR^{7'} ; with each R⁷ and R^{7'} independently as defined above for W.

Where J is -Q-Ar, Q is preferably a single bond, -O-, -S-, -N(R⁷)-, -(CH₂)ₘ-, -C(O)-, -CH(OH)-, -(CH₂)ₘO-, -(CH₂)ₘS-, -(CH₂)ₘN(R⁷)-, -O(CH₂)ₘ ,-CHX^{a}-, -CX^{a}₂-, -S-(CH₂)ₘ- and -N(R⁷)(CH₂)ₘ-, where m= 1-3, and X^{a} is halogen and
Ar is a 5- or 6-member aromatic structure. This aromatic structure of Ar
a) contains 0-2 members selected from the group consisting of nitrogen, oxygen and sulfur,
b) is optionally substituted by halogen, up to per-halo, and
c) is optionally substituted by Zₙ₁, wherein n1 is 0 to 3 and each Z is independently selected from the group consisting of -CN, -NO₂, -OR⁷, - SR⁷, -NR⁷R^{7'}, -NR⁷C(O)OR^{7'}, -NR⁷C(O)R^{7'}, with each R⁷ and R^{7'} independently as defined above for W, C₁₋₁₀ alkyl, C₁₋₁₀ alkoxy, C₂₋₁₀ alkenyl and C₁₋₁₀ alkenoyl halo substituted C₁₋₁₀ alkyl up to per halo, halo substituted C₁₋₁₀ alkoxy up to per halo, halosubstituted C₂₋₁₀ alkenyl up to per halo and halosubstituted C₁₋₁₀ alkenoyl up to per halo.

Where A is a substituted pyridyl, substituted quinolinyl or isoquinolinyl group, A is preferably substituted 1 to 3 times by 1 or more substituents selected from the group consisting of -CN, halogen, C₁-C₁₀ alkyl, C₁-C₁₀ alkoxy, -OH, up to per halo substituted C₁-C₁₀ alkyl, up to per halo substituted C₁-C₁₀ alkoxy or phenyl substituted by halogen up to per halo.

Where B is -L(ML¹)_{q}, L¹ can be substituted by the substituents -C(O)R^{a}, -C(NR^{a})R^{b}, -C(O)NR^{a}R^{b}, -SO₂NR^{a}R^{b}, -and -SO₂R^{a} wherein each R^{a} and R^{b} are independently hydrogen or a carbon based moiety of up to 24 carbon atoms, optionally containing heteroatoms selected from N, S and O, and optionally substituted by halogen.

R^{a} and R^{b} preferably are each, independently, C₁₋₁₀ alkyl, C₁₋₁₀ alkoxy, C₃₋₁₀ cycloalkyl having at least 5 cyclic members and 0-3 heteroatoms selected from N, S and O, C₂₋₁₀ alkenyl, C₁₋₁₀ alkenoyl, C₆₋₁₄ aryl, C₃₋₁₂ hetaryl having 1-3 heteroatoms selected from N, S and O, C₇₋₂₄ aralkyl, C₇₋₂₄ alkaryl, substituted C₁₋₁₀ alkyl, substituted C₁₋₁₀ alkoxy, substituted C₃₋₁₀ cycloalkyl having at least 5 cyclic members and 0-3 heteroatoms selected from N, S and O, substituted C₂₋₁₀ alkenyl, substituted C₁₋₁₀ alkenoyl, substituted C₆ -C₁₄ aryl, substituted C₃₋₁₂ hetaryl having at least 5 cyclic members and 1-3 heteroatoms selected from N, S and O, substituted C₇-₂₄ alkaryl or substituted C₇-C₂₄ aralkyl. Where R^{a} and/or R^{b} are a substituted group, they are substituted by halogen up to per halo hydroxy, C₁₋₁₀ alkyl, C₃₋₁₀ cycloalkyl having 0-3 heteroatoms selected from O, S and N, C₃₋₁₂ hetaryl having 1-3 heteroatoms selected from N, S and O, C₁₋₁₀ alkoxy,

C₆₋₁₂ aryl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)Rg.

R^{a} and R^{b} can also be -OSi(R_{f})₃ where R_{f} is hydrogen or a carbon based moiety of up to 24 carbon atoms optionally containing heteroatoms selected from N, S and O and optionally substituted by halogen, hydroxy and carbon based substituents of up to 24 carbon atoms, which optionally contain heteroatoms selected from N, S and O and are optionally substituted by halogen; or
b) bound together to form a 5-7 member heterocyclic structure of 1-3 heteroatoms selected from N, S and O, or a substituted 5-7 member heterocyclic structure of 1-3 heteroatoms selected from N, S and O substituted by halogen, hydroxy or carbon based substituents of up to 24 carbon atoms, which optionally contain heteroatoms selected from N, S and O and are optionally substituted by halogen; or
c) one of R^{a} or R^{b} can be -C(O)-, a C₁-C₅ divalent alkylene group or a substituted C₁-C₅ divalent alkylene group bound to the moiety L to form a cyclic structure with at least 5 members, wherein the substituents of the substituted C₁-C₅ divalent alkylene group are selected from the group consisting of halogen, hydroxy, and carbon based substituents of up to 24 carbon atoms, which optionally contain heteroatoms selected from N, S and O and are optionally substituted by halogen.

The carbon based moieties of R_{f} and the substituents on R^{a} and R^{b} include C₁₋₁₀ alkyl, C₁₋₁₀ alkyl, C₁₋₁₀ alkoxy, C₃-C₁₀ cycloalkyl having 0-3 heteroatoms selected from O, S and N, C₆₋₁₂ aryl, C₃-C₁₂ hetaryl having 1-3 heteroatoms selected from O, S and N, C₇₋₂₄ aralkyl, substituted C₁₋₁₀ alkyl, substituted C₁-C₁₀ alkoxy, substituted C₃-C₁₂ cycloalkyl having 0-3 heteroatoms selected from O, S and N, substituted C₃-C₁₂ heteraryl having 1-3 heteroatoms selected from O, S, and N, substituted C₆₋₁₂ aryl, and substituted C₇₋₂₄ alkaryl, where R_{f} is a substituted group it is substituted halogen up to per halo, hydroxy, C₁₋₁₀ alkyl, C₃₋₁₂ cycloalkyl having 0-3 heteroatoms selected from O, S and N, C₃₋₁₂ hetaryl having 1-3 heteroatoms selected from N, S and O, C₁₋₁₀ alkoxy, C₆₋₁₂ aryl, C_{y-}-C₂₄ alkaryl, C₇-C₂₄ aralkyl, C₁₋₆ halo substituted alkyl up to per halo alkyl, C₆-C₁₂ halo substituted aryl up to per halo aryl, C₃-C₁₂ halo substituted cycloalkyl having 0-3 heteroatoms selected from N, S and O, up to per halo cycloalkyl, halo substituted C₃-C₁₂ hetaryl up to per halo heteraryl, halo substituted C₇-C₂₄ aralkyl up to per halo aralkyl, halo substituted C₇-C₂₄ alkaryl up to per halo alkaryl, and -C(O)Rg;
where Rg is C₁₋₁₀ alkyl; -CN, -CO₂R_{d}, -OR_{d}, -SR_{d}, -NO₂, -C(O) Rₑ, -NR_{d}Rₑ, -NR_{d} C(O)ORₑ and -NR_{d}C(O)Rₑ, and R_{d} and Rₑ are independently selected from the group consisting of hydrogen, C₁₋₁₀, alkyl, C₁₋₁₀ alkoxy, C₃₋₁₀ cycloalkyl having 0-3 heteroatoms selected from O, N and S, C₆₋₁₂ aryl, C₃-C₁₂ hetaryl with 1-3 heteroatoms selected from O, N and S and C₇-C₂₄ aralkyl, C₇-C₂₄ alkaryl, up to per halo substituted C₁-C₁₀ alkyl, up to per halo substituted C₃-C₁₀ cycloalkyl having 0-3 heteroatoms selected from O, N and S, up to per halo substituted C₆-C₁₄ aryl, up to per halo substituted C₃-C₁₂ hetaryl having 1-3 heteroatoms selected from O, N, and S, halo substituted C₇-C₂₄ alkaryl up to per halo alkaryl, and up to per halo substituted C₇-C₂₄ aralkyl.

The bridging group M in the formula -L-(ML¹)_{q}, for B is preferably selected from the group consisting of -O-, -S-, -N(R⁷)-, -(CH₂)ₘ-, -C(O)-, -CH(OH)-, -(CH₂)ₘO-, -( CH₂)ₘS-, -( CH₂)ₘ N(R⁷)-, -O(CH₂)ₘ CHX^{a}-, -CX^{a}₂-, -S-(CH₂)ₘ-, -N(R⁷)(CH₂)ₘ- and -CR^{a}R^{b}- where m=1-3, X^{a} is hydrogen, R⁷, R^{a} and R^{b} are as defined above and q is 1. More preferably, M is -O-, -CH₂-, -S-, -NH-, -C(O)-, -O-CH₂- and -CH₂-O-.

The moieties L and L¹ in the formula -L-(ML¹)_{q} for B are typically each, independently, a substituted aryl moiety having at least 6 cyclic members, a substituted heterocyclic moiety having at least 5 cyclic members, an unsubstituted aryl moiety having at least 6 cyclic members or an unsubstituted heterocyclic moiety having at least 5 cyclic members. The heterocyclic and hetaryl moietes for L and L' typically have 1 to 4 members selected from the group of hetero atoms consisting of nitrogen, oxygen and sulfur with the balance of the hetaryl or heterocyclic moiety being carbon. More typical moieties for L¹ and L are selected from the group consisting of thiophene, substituted thiophene, phenyl, substituted phenyl, pyridinyl, substituted pyridinyl, pyrimidinyl and substituted pyrimidinyl.

Where L is substituted or L¹ is additionally substituted, the substituents are selected from the group consisting of halogen, up to per-halo, and Jn where n is 0-3, and J is as defined above.

Preferred compounds of Formula I include those wherein the cyclic structures of B and L bound directly to D are not substituted in the ortho position by-OH.

The invention provides hetaryl compounds containing nitrogen hetero-atoms of formula II

A'-D-B' (II),

wherein D is as defined above for formula I and A' is either a substituted t-butylpyridyl, unsubstituted t-butylpyridyl, substituted (trifluoromethyl)pyridyl, unsubstituted (trifluoromethyl)pyridyl, substituted isopropylpyridyl, unsubstituted sopropylpyridyl, substituted (2-methyl-2-butyl)pyridyl, unsubstituted (2-methyl-2-butyl)pyridyl, substituted (3-ethyl-3-pentyl)pyridyl, unsubstituted (3-ethyl-3-pentyl)pyridyl, substituted isoquinolinyl, unsubstituted isoquinolinyl or unsubstituted quinolinyl.
B' can be a substituted or unsubstituted 6 member cyclic aryl ring, at least a 5 member heterocylic ring or from 2-3 fused rings of up to 30 carbon atoms (aryl hetaryl or both).
B' also includes structures of formula III 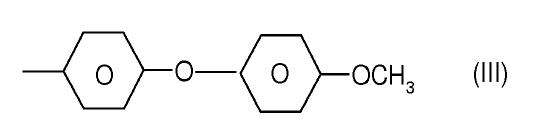 either substituted or unsubstituted, where A' is substituted or unsubstituted t-butylyridyl, (trifluoromethyl)pyridyl, isopropylpyridyl, (2-methyl-2-butyl)pyridyl or (3-ethyl-3-pentyl)pyridyl.

B' also includes structures of the formula IV 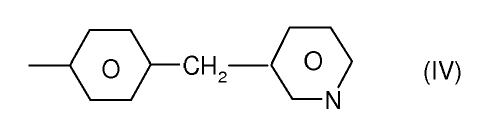 either substituted or unsubstituted where A' is a substituted isoquinolinyl, unsubsituted isoquinolinyl or unsubstituted quinolinyl group.

The substituents for the substituted groups of A' are as defined for A. Preferred substituents are selected from the group consisting of up to per halo substituted C₁₋₁₀ alkoxy, up to per halo substituted C₁₋₁₀ alkyl and C₃₋₁₀ heteroyclic moieties comprising 1 to 2 heteroatoms selected from the group consisting of nitrogen, oxygen and sulfur.

Where B' is a 6 member cyclic aryl ring, at least a 5 member heterocyclic ring or 2-3 fused rings of up to 30 carbon atoms, the substituents for B' are selected from the group consisting of halogen, up to per-halo-, and J¹ₙ where n=0-3 and each J¹ is independently selected from the group consisting of -CN, halogen, OH, -CO₂R⁷, C(O)N⁷R^{7'}, -C(O)-R⁷, NO₂, OR⁷, SR⁷, NR⁷R^{7'}, NR⁷C(O)OR^{7'}, NR⁷C(O)R^{7'}, C₁₋₁₀alkyl, C₁₋₁₀ alkoxy, C₂₋₁₀ alkenyl, C₁₋₁₀ alkenoyl, substituted C₁₋₁₀ alkyl, substituted C₁₋₁₀ alkoxy, substituted C₂₋₁₀ alkenyl, and substituted C₁₋₁₀ alkenoyl, with R⁷ and R^{7'} are, independently, as defined above.

When B' is of formula III or IV, the substituents are selected from the group consisting of -CN, halogen, OH, -NO₂, -OR⁷, -SR⁷, -NR⁷R^{7'}, -NR⁷C(O)OR^{7'}, C₁₋₁₀ alkyl, C₁₋₁₀ alkoxy, C₂₋₁₀ alkenyl, C₁₋₁₀ alkenoyl, substituted C₁₋₁₀ alkoxy, substituted C₂₋₁₀ alkenyl and substituted alkenoyl C₁₋₁₀, with R⁷ and R^{7'} as defined above.

R^{a} and R^{b} preferably are each, independently, C₁₋₁₀ alkyl, C₁₋₁₀ alkoxy, C₃₋₁₀ cycloalkyl having at least 5 cyclic members and 0-3 heteroatoms, C₂₋₁₀ alkenyl, C₁₋₁₀ alkenoyl, C₆₋₁₂ aryl, C₃₋₁₂ hetaryl having at least 5 cyclic members and 1-3 heteroatoms selected from N, S and O, C₇₋₂₄ aralkyl, C₇₋₂₄ alkaryl, substituted C₁₋₁₀ alkyl, substituted C₁₋₁₀ alkoxy, substituted C₃₋₁₀ cycloalkyl having at least 5 cyclic members and 0-3 heteroatoms selected from N, S and O, substituted C₂₋₁₀ alkenyl, substituted C₁₋₁₀ alkenoyl, substituted C₆ -C₁₄ aryl, substituted C₃₋₁₂ hetaryl having at least 5 cyclic members and 1-3 heteroatoms selected from N, S and O, substituted C₇₋₂₄ alkaryl or substituted C₇-C₂₄ aralkyl, where R^{a} and/or R^{b} are a substituted group, they are preferably substituted by halogen up to per halo.

Where B' is a substituted pyridyl, substituted quinolinyl or isoquinolinyl group, B' is preferably substituted 1 to 3 times by 1 or more substituents selected from the group consisting of -CN, halogen, C₁-C₁₀ alkyl, C₁-C₁₀ alkoxy, -OH, up to per halo substituted C₁-C₁₀ alkyl, up to per halo substituted C₁-C₁₀ alkoxy or phenyl substituted by halogen up to per halo.

In Formulae I, and II suitable hetaryl groups include, but are not limited to, 4-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 5-7 member atoms. For example, B can be 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or -5-yl, 1,2,4-triazol-1-, -3- or -5-yl, 1- or 5-tetrazolyl, 1,2,3-oxadiazol-4- or -5-yl, 1,2,4-oxadiazol-3- or -5-yl, 1,3,4-thiadiazol-2- or -5-yl, 1,2,4-oxadiazol-3- or -5-yl, 1,3,4-thiadiazol-2- or -5-yl, 1,3,4-thiadiazol-3- or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8-isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl, etc. For example, B can be 4-methyl-phenyl, 5-methyl-2-thienyl, 4-methyl-2-thienyl, 1-methyl-3-pyrryl, 1-methyl-3-pyrazolyl, 5-methyl-2-thiazolyl or 5-methyl-1,2,4-thiadiazol-2-yl.

Suitable alkyl groups and alkyl portions of groups, e.g., alkoxy, etc. throughout include methyl, ethyl, propyl, butyl, etc., including all straight-chain and branched isomers such as isopropyl, isobutyl, *sec*-butyl, *tert*-butyl, etc.

Suitable aryl groups which do not contain heteroatoms include, for example, phenyl and 1- and 2-naphthyl.

The term "cycloalkyl", as used herein, refers to cyclic structures with or without alkyl substituents such that, for example, "C₄ cycloalkyl" includes methyl substituted cyclopropyl groups as well as cyclobutyl groups. The term "cycloalkyl", as used herein also includes saturated heterocyclic groups.

Suitable halogen groups include F, Cl, Br, and/or I, from one to per-substitution (i.e. all H atoms on a group replaced by a halogen atom) being possible where an alkyl group is substituted by halogen, mixed substitution of halogen atom types also being possible on a given moiety.

The present invention is also directed to pharmaceutically acceptable salts of formula II. Suitable pharmaceutically acceptable salts are well known to those skilled in the art and include basic salts of inorganic and organic acids, such as hydrochloric acid, hydrobromic acid, sulphuric acid, phosphoric acid, methanesulphonic acid, trifluoromethanesulfonic acid, benzenesulphonic acid, *p*-toluenesulfonic acid, 1-naphthalenesulfonic acid, 2-naphthalenesulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid. In addition, pharmaceutically acceptable salts include acid salts of inorganic bases, such as salts containing alkaline cations (e.g., Li⁺ Na⁺ or K⁺), alkaline earth cations (e.g., Mg⁺² , Ca⁺² or Ba⁺²), the ammonium cation, as well as acid salts of organic bases, including aliphatic and aromatic substituted ammonium, and quaternary ammonium cations, such as those arising from protonation or peralkylation of triethylamine, *N,N*-diethylamine, *N,N*-dicyclohexylamine, lysine, pyridine, *N,N*-dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

A number of the compounds of Formulae I and II possess asymmetric carbons and can therefore exist in racemic and optically active forms. Methods of separation of enantiomeric and diastereomeric mixtures are well known to one skilled in the art. The present invention encompasses any racemic or optically active form of compounds described in Formula II which possess progesterone receptor binding activity.

### General Preparative Methods

The compounds of Formulae I and II may be prepared by the use of known chemical reactions and procedures, some from starting materials which are commercially available. Nevertheless, general preparative methods are provided below to aid one skilled in the art in synthesizing these compounds, with more detailed examples being provided in the Experimental section which follows.

Substituted and unsubstituted aminoquinolines, aminoisoquinolines and aminopyridines may be prepared using standard methods (see, for example: A.R. Katritzky et al. (Eds.). Comprehensive Heterocyclic Chemistry II, Vol. 5. M.H. Palmer. Heterocyclic Compounds; Arnold Ltd., London (1967). C.K. Esser et al. WO 96/18616. C.J. Donahue et al. Inorg. Chem. 30, 1991, 1588. E. Cho et al. WO 98/00402. A. Cordi et al. Bioorg. Med. Chem.. 3, 1995, 129). In addition, many aminoquinolines, aminoisoquinolines and aminopyridines are commercially available.

Substituted anilines may be generated using standard methods (March. Advanced Organic Chemistry, 3rd Ed.; John Wiley: New York (1985). Larock. Comprehensive Organic Transformations; VCH Publishers: New York (1989)). As shown in Scheme I, aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. Hydrogenation Methods; Academic Press: London, UK (1985)). Nitroaryls may also be directly reduced using a strong hydride source, such as LiA1H₄ (Seyden-Penne. Reductions by the Alumino- and Borohydrides in Organic Synthesis; VCH Publishers: New York (1991)), or using a zero valent metal, such as Fe, Sn or Ca, often in acidic media. Many methods exist for the synthesis of nitroaryls (March. Advanced Organic Chemistry, 3rd Ed.; John Wiley: New York (1985). Larock. Comprehensive Organic Transformations; VCH Publishers: New York (1989)).

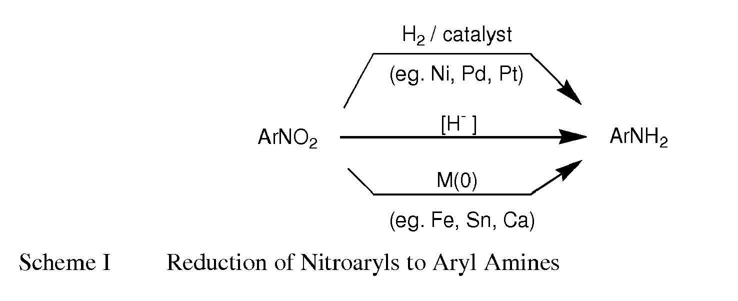

Nitroaryls are commonly formed by electrophilic aromatic nitration using HNO₃, or an alternative NO₂⁺ source. Nitroaryls may be further elaborated prior to reduction. Thus, nitroaryls substituted with 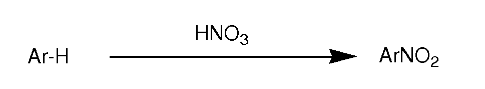 potential leaving groups (eg. F, Cl, Br, etc.) may undergo substitution reactions on treatment with nucleophiles, such as thiolate (exemplified in Scheme II) or phenoxide. Nitroaryls may also undergo Ullman-type coupling reactions (Scheme II).

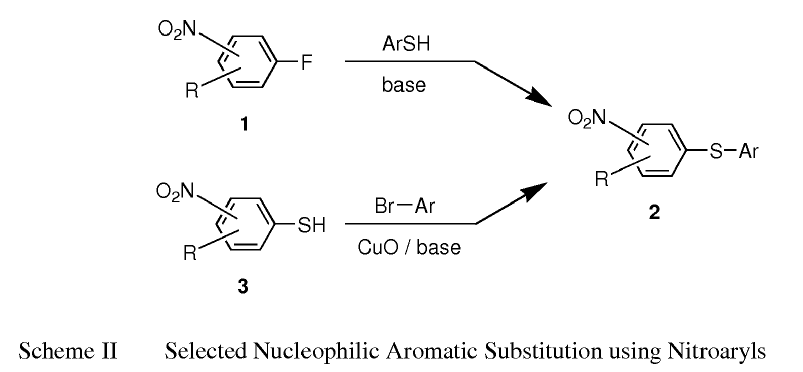

Nitroaryls may also undergo transition metal mediated cross coupling reactions. For example, nitroaryl electrophiles, such as nitroaryl bromides, iodides or triflates, undergo palladium mediated cross coupling reactions with aryl nucleophiles, such as arylboronic acids (Suzuki reactions, exemplified below), aryltins (Stille reactions) or arylzincs (Negishi reaction) to afford the biaryl (**5**).

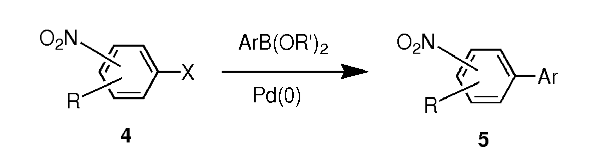

Either nitroaryls or anilines may be converted into the corresponding arenesulfonyl chloride (**7**) on treatment with chlorosulfonic acid. Reaction of the sulfonyl chloride with a fluoride source, such as KF then affords sulfonyl fluoride (**8**). Reaction of sulfonyl fluoride **8** with trimethylsilyl trifluoromethane in the presence of a fluoride source, such as tris(dimethylamino)sulfonium difluorotrimethylsiliconate (TASF) leads to the corresponding trifluoromethylsulfone (**9**). Alternatively, sulfonyl chloride **7** may be reduced to the arenethiol (**10**), for example with zinc amalgum. Reaction of thiol **10** with CHClF₂ in the presence of base gives the difluoromethyl mercaptan (**11**), which may be oxidized to the sulfone (**12**) with any of a variety of oxidants, including CrO₃-acetic anhydride (Sedova et al. Zh. Org. Khim. 1970, 6, (568).

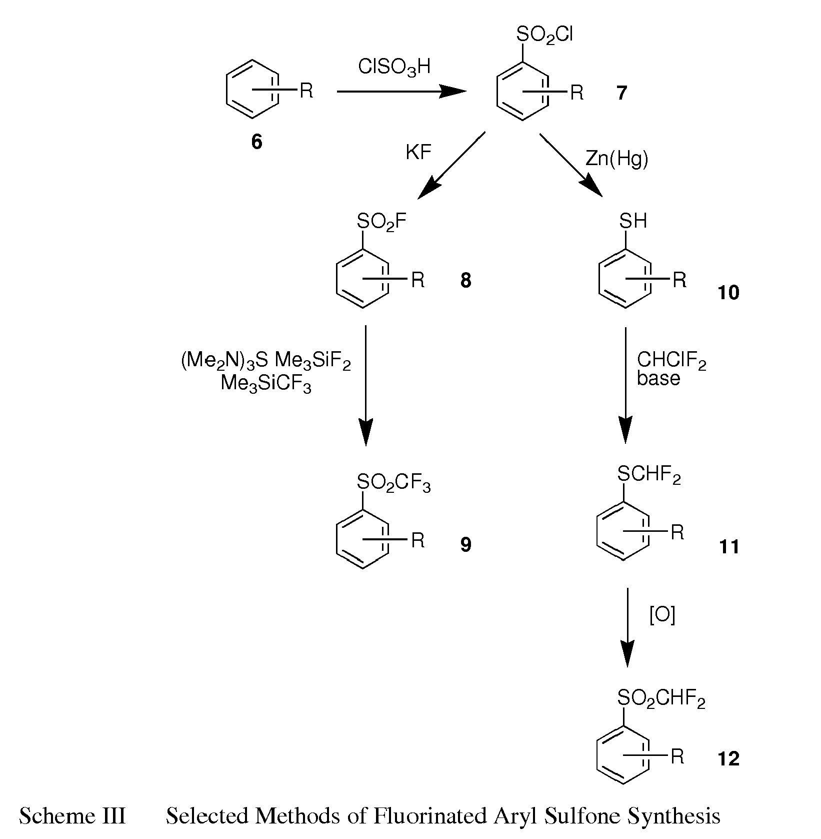

As shown in Scheme IV, non-symmetrical urea formation may involve reaction of an aryl isocyanate (**14**) with an aryl amine (**13**). The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or *N*,*N*'-carbonyldiimidazole (CDI). The isocyanate may also be derived from a heterocyclic carboxylic acid derivative, such as an ester, an acid halide or an anhydride by a Curtius-type rearrangement. Thus, reaction of acid derivative **16** with an azide source, followed by rearrangement affords the isocyanate. The corresponding carboxylic acid (**17**) may also be subjected to Curtius-type rearrangements using diphenylphosphoryl azide (DPPA) or a similar reagent.

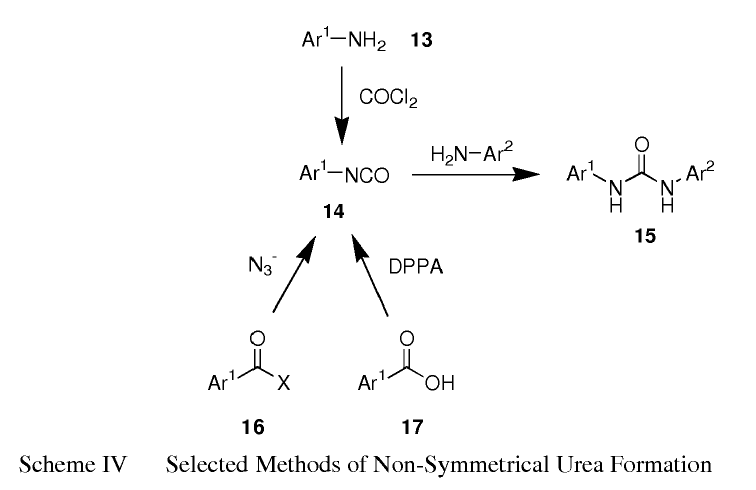

Finally, ureas may be further manipulated using methods familiar to those skilled in the art.

The invention also includes pharmaceutical compositions including a compound of Formula I, and a physiologically acceptable carrier.

The compounds may be administered orally, dermally, parenterally, by injection, by inhalation or spray, or sublingually, rectally or vaginally in dosage unit formulations. The term 'administration by injection' includes intravenous, intraarticular, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl, *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oil phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal or vaginal temperature and will therefore melt in the rectum or vagina to release the drug. Such materials include cocoa butter and polyethylene glycols.

Compounds of the invention may also be administered transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures one or more materials selected from lower alcohols, lower ketones , lower carboxylic acid esters, polar ethers, lower hydrocarbons, halogenated hydrocarbons.

Suitable penetration enhancing materials for transdermal delivery systems are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, *n*-butyl, *sec*-butyl isobutyl *tert*-butyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated dicarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene coploymers, and natural and synthetic rubbers. Cellulose ethers, derivatized polyethylenes, and silicates may also be used as matrix components. Additional additives, such as viscous resins or oils may be added to increase the viscosity of the matrix.

For all regimens of use disclosed herein for compounds of Formulae I and II, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily rectal dosage regimen will preferably be from 0.01 to 200 mg/Kg of total body weight. The daily vaginal dosage regimen will preferably be from 0.01 to 200 mg/Kg of total body weight. These daily dosages can be administered incrementally during the day, on a weekly basis on a biweekly basis or longer periods. Long term dosages typically range from 100-600 mg/kg of total body weight and preferably range from 100-400 mg/kg of total body weight.

Dosages for oral, vaginal and rectal administration and administration by injection can range from 0.01 mg - 600 mg/kg of total body weight.

The daily topical dosage regimen will preferably be from 0.1 to 200 mg administered between one to four times daily. The transdermal concentration will preferably be that required to maintain a daily dose of from 0.01 to 200 mg/Kg. The daily inhalation dosage regimen will preferably be from 0.01 to 10 mg/Kg of total body weight.

It will be appreciated by those skilled in the art that the particular method of administration will depend on a variety of factors, all of which are considered routinely when administering therapeutics. It will also be understood, however, that the specific dose level for any given patient will depend upon a variety of factors, including, but not limited to the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, ie., the mode of treatment and the daily or weekly number of doses of a compound of Formulae I or II or a pharmaceutically acceptable salt thereof given for a defined number of days, can be ascertained by those skilled in the art using conventional treatment tests.

The entire disclosure of all applications, patents and publications cited above and below are hereby incorporated by reference, including provisional application Serial No. 60/115,878 filed January 13, 1999 and non-provisional applications
Serial No. 09/778,039, filed February 7, 2001, and
Serial No. 09/257,265, filed February 25, 1999 and
Serial No. 09/425,229, filed October 22, 1999.

The compounds of formulae I and II are producible from known compounds (or from starting materials which, in turn, are producible from known compounds), e.g., through the general preparative methods shown below. The activity of a given compound to inhibit raf kinase can be routinely assayed, e.g., according to procedures disclosed below. The following examples are for illustrative purposes only and are not intended, nor should they be construed to limit the invention in any way.

### EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg. Unless otherwise stated, the term 'under high vacuum' refers to a vacuum of 0.4 - 1.0 mmHg.

All temperatures are reported in degrees Celsius (°C). Unless otherwise indicated, all parts and percentages are by weight.

Commercial grade reagents and solvents were used without further purification. *N-*cyclohexyl-*N*'-(methylpolystyrene)carbodiimide was purchased from Calbiochem-Novabiochem Corp. 5-(Trifluoromethyl)-2-aminopyridine, 3-aminoqunioline, 3-aminoisoquinoline, 1-(4-methylpiperazinyl)-3-aminoisoquinoline, ethyl 4-isocyanatobenzoate, *N*-acetyl-4-chloro-2-methoxy-5-(trifluoromethyl)aniline, 4-(4-nitrobenzyl)pyridine, 4-phenoxyaniline, 4-(4-methylphenoxy)aniline, 4-(4-chlorophenoxy)aniline and 4-chloro-3-(trifluoromethyl)phenyl isocyanate were purchased and used without further purification. Syntheses of 2-amino-4-*tert*-butylpyridine (C.K. Esser et al. WO 96/18616; C.J. Donahue et al. Inorg. Chem. 30, 1991, 1588), 3-amino-2-methoxyquinoline (E. Cho et al. WO 98/00402; A. Cordi et al. EP 542,609; IBID Bioorg. Med. Chem.. 3, 1995, 129), 4-(3-carbamoylphenoxy)-1-nitrobenzene (K. Ikawa Yakugaku Zasshi 79, 1959, 760; Chem. Abstr. 53, 1959, 12761b), 4-[(4-methoxyphenyl)methylamino]aniline (P. Brenneisen et al. US 3,755,406; *IBID* US 3,839,582; *IBID* DE 1,935,388), 4-(4-pyridylcarbonyl)aniline (M.L. Carmello et al. Pestic. Sci. 45, 1995, 227), 3-*tert*-butylphenyl isocyanate (O. Rohr et al. DE 2,436,108) and 2-methoxy-5-(trifluoromethyl)phenyl isocyanate (K. Inukai et al. JP 42,025,067; IBID Kogyo Kagaku Zasshi 70, 1967, 491) have previously been described.

Thin-layer chromatography (TLC) was performed using Whatman^{®} pre-coated glass-backed silica gel 60A F-254 250 µm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science^{®} silica gel.

Melting points (mp) were determined using a Thomas-Hoover melting point apparatus or a Mettler FP66 automated melting point apparatus and are uncorrected. Fourier transform infrared sprectra were obtained using a Mattson 4020 Galaxy Series spectrophotometer. Proton (¹H) nuclear magnetic resonance (NMR) spectra were measured with a General Electric GN-Omega 300 (300 MHz) spectrometer with either Me₄Si (* 0.00) or residual protonated solvent (CHCl₃ * 7.26; MeOH * 3.30; DMSO * 2.49) as standard. Carbon (¹³C) NMR spectra were measured with a General Electric GN-Omega 300 (75 MHz) spectrometer with solvent (CDCl₃ * 77.0; MeOD-d₃; * 49.0; DMSO-d₆ * 39.5) as standard. Low resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250 °C. Electron impact ionization was performed with electron energy of 70 eV and a trap current of 300 µA. Liquid-cesium secondary ion mass spectra (FAB-MS), an updated version of fast atom bombardment were obtained using a Kratos Concept 1-H spectrometer. Chemical ionization mass spectra (CI-MS) were obtained using a Hewlett Packard MS-Engine (5989A) with methane or ammonia as the reagent gas (1x10⁻⁴ torr to 2.5x10⁻⁴ torr). The direct insertion desorption chemical ionization (DCI) probe (Vaccumetrics, Inc.) was ramped from 0-1.5 amps in 10 sec and held at 10 amps until all traces of the sample disappeared (~1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an HP-1 methyl silicone column (0.33 mM coating; 25 m x 0.2 mm) and a Hewlett Packard 5971 Mass Selective Detector (ionization energy 70 eV). Elemental analyses were conducted by Robertson Microlit Labs, Madison NJ.

All compounds displayed NMR spectra, LRMS and either elemental analysis or HRMS consistant with assigned structures.

### List of Abbreviations and Acronyms:

- AcOH: acetic acid
- anh: anhydrous
- atm: atmosphere(s)
- BOC: *tert*-butoxycarbonyl
- CDI: 1,1'-carbonyl diimidazole
- conc: concentrated
- dec: decomposition
- DMAC: *N*,*N*-dimethylacetamide
- DMPU: 1,3-dimethyl-3,4,5,6-tetrahydro-2(1H)-pyrimidinone
- DMF: *N*,*N*-dimethylformamide
- DMSO: dimethylsulfoxide
- DPPA: diphenylphosphoryl azide
- EDCI: 1-(3-dimethylaminopropyl)-3-ethylcarbodiimide
- EtOAc: ethyl acetate
- EtOH: ethanol (100%)
- Et₂O: diethyl ether
- Et₃N: triethylamine
- HOBT: 1-hydroxybenzotriazole
- *m*-CPBA: 3-chloroperoxybenzoic acid
- MeOH: methanol
- pet. ether: petroleum ether (boiling range 30-60 °C)
- THF: tetrahydrofuran
- TFA: trifluoroacetic acid
- Tf: trifluoromethanesulfonyl

### A. General Methods for Synthesis of Substituted Anilines

### A1. General Method for Substituted Aniline Formation via Hydrogenation of a Nitroarene

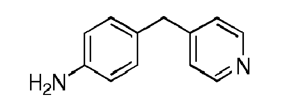

**4-(4-Pyridinylmethyl)aniline**: To a solution of 4-(4-nitrobenzyl)pyridine (7.0 g, 32.68 mmol) in EtOH (200 mL) was added 10% Pd/C (0.7 g) and the resulting slurry was shaken under a H₂ atmosphere (50 psi) using a Parr shaker. After 1 h, TLC and ¹H-NMR of an aliquot indicated complete reaction. The mixture was filtered through a short pad of Celite^{®}. The filtrate was concentrated *in vacuo* to afford a white solid (5.4 g, 90%): ¹H-NMR (DMSO-d₆) * 3.74 (s, 2H), 4.91 (br s, 2H), 6.48 (d, *J*=8.46 Hz, 2H), 6.86 (d, *J*=8.09 Hz, 2H), 7.16 (d, *J*=5.88 Hz, 2H), 8.40 (d, *J*=5.88 Hz, 2H); EI-MS *m*/*z* 184 (M⁺). This material was used in urea formation reactions without further purification.

### A2. General Method for Substituted Aniline Formation via Dissolving Metal Reduction of a Nitroarene

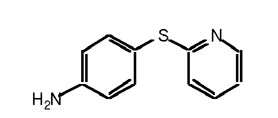

**4-(2-Pyridinylthio)aniline**: To a solution of 4-(2-pyridinylthio)-1-nitrobenzene (Menai ST 3355A; 0.220 g, 0.95 mmol) and H₂O (0.5 mL) in AcOH (5 mL) was added iron powder (0.317 g, 5.68 mmol) and the resulting slurry stirred for 16 h at room temp. The reaction mixture was diluted with EtOAc (75 mL) and H₂O (50 mL), basified to pH 10 by adding solid K₂CO₃ in portions (***Caution***: foaming). The organic layer was washed with a saturated NaCl solution, dried (MgSO₄), concentrated *in vacuo.* The residual solid was purified by MPLC (30% EtOAc/70% hexane) to give the desired product as a thick oil (0.135 g, 70%): TLC (30% EtOAc/70% hexanes) R*_{f}* 0.20.

### A3a. General Method for Substituted Aniline Formation via Nitroarene Formation Through Nucleophilic Aromatic Substitution, Followed by Reduction

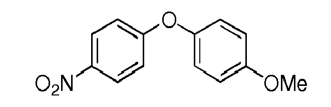

**Step 1. 1-Methoxy-4-(4-nitrophenoxy)benzene**: To a suspension of NaH (95%, 1.50 g, 59 mmol) in DMF (100 mL) at room temp. was added dropwise a solution of 4-methoxyphenol (7.39 g, 59 mmol) in DMF (50 mL). The reaction was stirred 1 h, then a solution of 1-fluoro-4-nitrobenzene (7.0 g, 49 mmol) in DMF (50 mL) was added dropwise to form a dark green solution. The reaction was heated at 95 °C overnight, then cooled to room temp., quenched with H₂O, and concentrated *in vacuo.* The residue was partitioned between EtOAc (200 mL) and H₂O (200 mL). The organic layer was sequentially washed with H₂O (2 x 200 mL), a saturated NaHCO₃ solution (200 mL), and a saturated NaCl solution (200 mL), dried (Na₂SO₄), and concentrated *in vacuo.* The residue was triturated (Et₂O/hexane) to afford 1-methoxy-4-(4-nitrophenoxy)benzene (12.2 g, 100%): ¹H-NMR (CDCl₃) * 3.83 (s, 3H), 6.93-7.04 (m, 6H), 8.18 (d, *J*=9.2 Hz, 2H); EI-MS *m*/*z* 245 (M⁺).

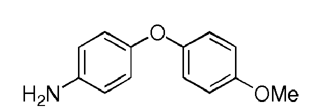

**Step 2. 4-(4-Methoxyphenoxy)aniline**: To a solution of 1-methoxy-4-(4-nitrophenoxy)benzene (12.0 g, 49 mmol) in EtOAc (250 mL) was added 5% Pt/C (1.5 g) and the resulting slurry was shaken under a H₂ atmosphere (50 psi) for 18 h. The reaction mixture was filtered through a pad of Celite^{®} with the aid of EtOAc and concentrated *in vacuo* to give an oil which slowly solidified (10.6 g, 100%): ¹H-NMR (CDCl₃) * 3.54 (br s, 2H), 3.78 (s, 3H), 6.65 (d, *J*=8.8 Hz, 2H), 6.79-6.92 (m, 6H); EI-MS *m*/*z* 215 (M⁺).

### A3b. General Method for Substituted Aniline Formation via Nitroarene Formation Through Nucleophilic Aromatic Substitution, Followed by Reduction

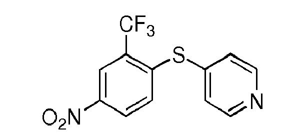

**Step 1. 3-(Trifluoromethyl)-4-(4-pyridinylthio)nitrobenzene**: A solution of 4-mercaptopyridine (2.8 g, 24 mmoles), 2-fluoro-5-nitrobenzotrifluoride (5 g, 23.5 mmoles), and potassium carbonate (6.1 g, 44.3 mmoles) in anhydrous DMF (80 mL) was stirred at room temperature and under argon overnight. TLC showed complete reaction. The mixture was diluted with Et₂O (100 mL) and water (100 mL) and the aqueous layer was back-extracted with Et₂O (2 x 100 mL). The organic layers were washed with a saturated NaCl solution (100 mL), dried (MgSO₄), and concentrated under reduced pressure. The solid residue was triturated with Et₂O to afford the desired product as a tan solid (3.8 g, 54%): TLC (30% EtOAc/70% hexane) R*_{f}* 0.06; ¹H-NMR (DMSO-d₆) * 7.33 (dd, *J*=1.2, 4.2 Hz, 2H), 7.78 (d, *J*=8.7 Hz, 1H), 8.46 (dd, *J*=2.4, 8.7Hz, 1H), 8.54-8.56 (m, 3H).

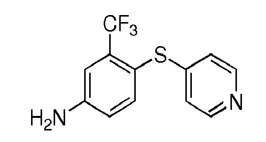

**Step 2. 3-(Trifluoromethyl)-4-(4-pyridinylthio)anilin**e: A slurry of 3-trifluoromethyl-4-(4-pyridinylthio)nitrobenzene (3.8 g, 12.7 mmol), iron powder (4.0 g, 71.6 mmol), acetic acid (100 mL), and water (1 mL) were stirred at room temp. for 4 h. The mixture was diluted with Et₂O (100 mL) and water (100 mL). The aqueous phase was adjusted to pH 4 with a 4 N NaOH solution. The combined organic layers were washed with a saturated NaCl solution (100 mL), dried (MgSO₄), and concentrated under reduced pressure. The residue was filtered through a pad of silica (gradient from 50% EtOAc/50% hexane to 60% EtOAc/40% hexane) to afford the desired product (3.3 g): TLC (50% EtOAc/50% hexane) R*_{f}* 0.10; ¹H-NMR (DMSO-d₆) * 6.21 (s, 2H), 6.84-6.87 (m, 3H), 7.10 (d, *J*=2.4 Hz, 1H), 7.39 (d, *J*=8.4 Hz, 1H), 8.29 (d, *J*=6.3 Hz, 2H).

### A3c. General Method for Substituted Aniline Formation via Nitroarene Formation Through Nucleophilic Aromatic Substitution, Followed by Reduction

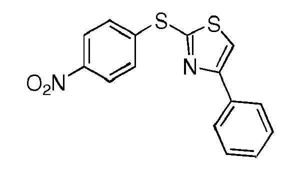

**Step 1. 4-(2-(4-Phenyl)thiazolyl)thio-1-nitrobenzene**: A solution of 2-mercapto-4-phenylthiazole (4.0 g, 20.7 mmoles) in DMF (40 mL) was treated with 1-fluoro-4-nitrobenzene (2.3 mL, 21.7 mmoles) followed by K₂CO₃ (3.18 g, 23 mmol), and the mixture was heated at approximately 65 °C overnight. The reaction mixture was then diluted with EtOAc (100 mL), sequentially washed with water (100 mL) and a saturated NaCl solution (100 mL), dried (MgSO₄) and concentrated under reduced pressure. The solid residue was triturated with a Et₂O/hexane solution to afford the desired product (6.1 g): TLC (25% EtOAc/75% hexane) R*_{f}* 0.49; ¹H-NMR (CDCl₃) * 7.35-7.47 (m, 3H), 7.58-7.63 (m, 3H), 7.90 (d, *J*=6.9 Hz, 2H), 8.19 (d, *J*=9.0 Hz, 2H).

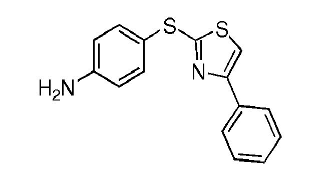

**Step 2. 4-(2-(4-Phenyl)thiazolyl)thioaniline**: 4-(2-(4-Phenyl)thiazolyl)thio-1-nitrobenzene was reduced in a manner analagous to that used in the preparation of 3-(trifluoromethyl)-4-(4-pyridinylthio)aniline: TLC (25% EtOAc/75% hexane) R*_{f}* 0.18; ¹H-NMR (CDCl₃) * 3.89 (br s, 2H), 6.72-6.77 (m, 2H), 7.26-7.53 (m, 6H), 7.85-7.89 (m, 2H).

### A3d. General Method for Substituted Aniline Formation via Nitroarene Formation Through Nucleophilic Aromatic Substitution, Followed by Reduction

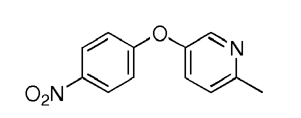

**Step 1. 4-(6-Methyl-3-pyridinyloxy)-1-nitrobenzene**: To a solution of 5-hydroxy-2-methylpyridine (5.0 g, 45.8 mmol) and 1-fluoro-4-nitrobenzene (6.5 g, 45.8 mmol) in anh DMF (50 mL) was added K₂CO₃ (13.0 g, 91.6 mmol) in one portion. The mixture was heated at the reflux temp. with stirring for 18 h and then allowed to cool to room temp. The resulting mixture was poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo* to afford the desired product (8.7 g, 83%). This material was carried to the next step without further purification.

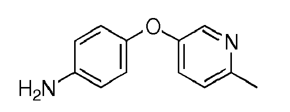

**Step 2. 4-(6-Methyl-3-pyridinyloxy)aniline**: A solution of 4-(6-methyl-3-pyridinyloxy)-1-nitrobenzene (4.0 g, 17.3 mmol) in EtOAc (150 mL) was added to 10% Pd/C (0.500 g, 0.47 mmol) and the resulting mixture was placed under a H₂ atmosphere (balloon) and was allowed to stir for 18 h at room temp. The mixture was then filtered through a pad of Celite^{®} and concentrated *in vacuo* to afford the desired product as a tan solid (3.2 g, 92%): EI-MS *m*/*z* 200 (M⁺).

### A3e. General Method for Substituted Aniline Formation via Nitroarene Formation Through Nucleophilic Aromatic Substitution, Followed by Reduction

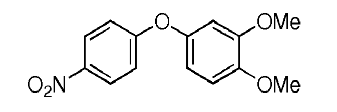

**Step 1. 4-(3,4-Dimethoxyphenoxy)-1-nitrobenzene**: To a solution of 3,4-dimethoxyphenol (1.0 g, 6.4 mmol) and 1-fluoro-4-nitrobenzene (700 µL, 6.4 mmol) in anh DMF (20 mL) was added K₂CO₃ (1.8 g, 12.9 mmol) in one portion. The mixture was heated at the reflux temp with stirring for 18 h and then allowed to cool to room temp. The mixture was then poured into water (100 mL) and extracted with EtOAc (3 x 100 mL). The combined organics were sequentially washed with water (3 x 50 mL) and a saturated NaCl solution (2 x 50 mL), dried (Na₂SO₄), and concentrated *in vacuo* to afford the desired product (0.8 g, 54%). The crude product was carried to the next step without further purification.

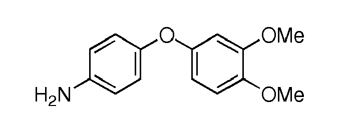

**Step 2. 4-(3,4-Dimethoxyphenoxy)aniline**: A solution of 4-(3,4-dimethoxy-phenoxy)-1-nitrobenzene (0.8 g, 3.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a white solid (0.6 g, 75%): EI-MS *m*/*z* 245 (M⁺).

### A3f. General Method for Substituted Aniline Formation via Nitroarene Formation Through Nucleophilic Aromatic Substitution, Followed by Reduction

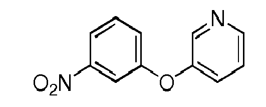

**Step 1. 3-(3-Pyridinyloxy)-1-nitrobenzene**: To a solution of 3-hydroxypyridine (2.8 g, 29.0 mmol), 1-bromo-3-nitrobenzene (5.9 g, 29.0 mmol) and copper(I) bromide (5.0 g, 34.8 mmol) in anh DMF (50 mL) was added K₂CO₃ (8.0 g, 58.1 mmol) in one portion. The resulting mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo.* The resulting oil was purified by flash chromatography (30% EtOAc/70% hexane) to afford the desired product (2.0 g, 32 %). This material was used in the next step without further purification.

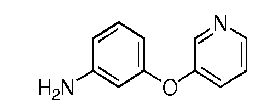

**Step 2. 3-(3-Pyridinyloxy)aniline**: A solution of 3-(3-pyridinyloxy)-1-nitrobenzene (2.0 g, 9.2 mmol) in EtOAc (100 mL) was added to 10% Pd/C (0.200 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a red oil (1.6 g, 94%): EI-MS *m*/*z* 186 (M⁺).

### A3g. General Method for Substituted Aniline Formation via Nitroarene Formation Through Nucleophilic Aromatic Substitution, Followed by Reduction

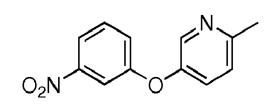

**Step 1. 3-(5-Methyl-3-pyridinyloxy)-1-nitrobenzene**: To a solution of 3-hydroxy-5-methylpyridine (5.0 g, 45.8 mmol), 1-bromo-3-nitrobenzene (12.0 g, 59.6 mmol) and copper(I) iodide (10.0 g, 73.3 mmol) in anh DMF (50 mL) was added K₂CO₃ (13.0 g, 91.6 mmol) in one portion. The mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo.* The resulting oil was purified by flash chromatography (30% EtOAc/70% hexane) to afford the desired product (1.2 g, 13%).

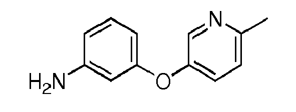

**Step 2. 3-(5-Methyl-3-pyridinyloxy)-1-nitrobenzene**: A solution of 3-(5-methyl-3-pyridinyloxy)-1-nitrobenzene (1.2 g, 5.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a red oil (0.9 g, 86%): CI-MS *m*/*z* 201 ((M+H)⁺).

### A3h. General Method for Substituted Aniline Formation via Nitroarene Formation Through Nucleophilic Aromatic Substitution, Followed by Reduction

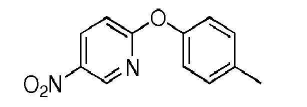

**Step 1. 5-Nitro-2-(4-methylphenoxy)pyridine**: To a solution of 2-chloro-5-nitropyridine (6.34 g, 40 mmol) in DMF (200 mL) were added of 4-methylphenol (5.4 g, 50 mmol, 1.25 equiv) and K₂CO₃ (8.28 g, 60 mmol, 1.5 equiv). The mixture was stirred overnight at room temp. The resulting mixture was treated with water (600 mL) to generate a precipitate. This mixture was stirred for 1 h, and the solids were separated and sequentially washed with a 1 N NaOH solution (25 mL), water (25 mL) and pet ether (25 mL) to give the desired product (7.05 g, 76%): mp 80-82 °C; TLC (30% EtOAc/70% pet ether) R*_{f}* 0.79; ¹H-NMR (DMSO-d₆) * 2.31 (s, 3H), 7.08 (d, *J*=8.46 Hz, 2H), 7.19 (d, *J*=9.20 Hz, 1H), 7.24 (d, *J*=8.09 Hz, 2H), 8.58 (dd, *J*=2.94, 8.82 Hz, 1H), 8.99 (d, *J*=2.95 Hz, 1H); FAB-MS *m*/*z* (rel abundance) 231 ((M+H)⁺), 100%).

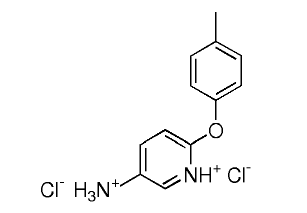

**Step 2. 5-Amino-2-(4-methylphenoxy)pyridine Dihydrochloride**: A solution 5-nitro-2-(4-methylphenoxy)pyridine (6.94 g, 30 mmol, 1 eq) and EtOH (10 mL) in EtOAc (190 mL) was purged with argon then treated with 10% Pd/C (0.60 g). The reaction mixture was then placed under a H₂ atmosphere and was vigorously stirred for 2.5 h. The reaction mixture was filtered through a pad of Celite^{®}. A solution of HCl in Et₂O was added to the filtrate was added dropwise. The resulting precipitate was separated and washed with EtOAc to give the desired product (7.56 g, 92%): mp 208-210 °C (dec); TLC (50% EtOAc/50% pet ether) R*_{f}* 0.42; ¹H-NMR (DMSO-d₆) 2.25 (s, 3H), 6.98 (d, *J*=8.45 Hz, 2H), 7.04 (d, *J*=8.82 Hz, 1H), 7.19 (d, *J*=8.09 Hz, 2H), 8.46 (dd, *J*=2.57, 8.46 Hz, 1H), 8.63 (d, *J*=2.57 Hz, 1H); EI-MS *m*/*z* (rel abundance) (M⁺, 100%).

### A3i. General Method for Substituted Aniline Formation via Nitroarene Formation Through Nucleophilic Aromatic Substitution, Followed by Reduction

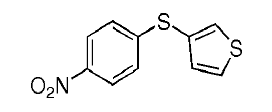

**Step 1. 4-(3-Thienylthio)-1-nitrobenzene**: To a solution of 4-nitrothiophenol (80%pure; 1.2 g, 6.1 mmol), 3-bromothiophene (1.0 g, 6.1 mmol) and copper(II) oxide (0.5 g, 3.7 mmol) in anhydrous DMF (20 mL) was added KOH (0.3 g, 6.1 mmol), and the resulting mixture was heated at 130 °C with stirring for 42 h and then allowed to cool to room temp. The reaction mixture was then poured into a mixture of ice and a 6N HCl solution (200 mL) and the resulting aqueous mixture was extracted with EtOAc (3 x 100 mL). The combined organic layers were sequentially washed with a 1M NaOH solution (2 x 100 mL) and a saturated NaCl solution (2 x 100 mL), dried (MgSO₄), and concentrated *in vacuo.* The residual oil was purified by MPLC (silica gel; gradient from 10% EtOAc/90% hexane to 5% EtOAc/95% hexane) to afford of the desired product (0.5 g, 34%). GC-MS *m*/z 237 (M⁺).

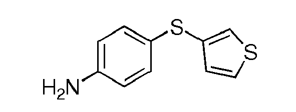

**Step 2. 4-(3-Thienylthio)aniline**: 4-(3-Thienylthio)-1-nitrobenzene was reduced to the aniline in a manner analogous to that described in Method A1.

### A3j. General Method for Substituted Aniline Formation via Nitroarene Formation Through Nucleophilic Aromatic Substitution, Followed by Reduction

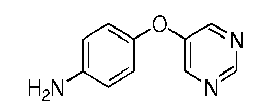

**4-(5-Pyrimininyloxy)aniline**: 4-Aminophenol (1.0 g, 9.2 mmol) was dissolved in DMF (20 mL) then 5-bromopyrimidine (1.46 g, 9.2 mmol) and K₂CO₃ (1.9 g, 13.7 mmol) were added. The mixture was heated to 100 °C for 18 h and at 130 °C for 48 h at which GC-MS analysis indicated some remaining starting material. The reaction mixture was cooled to room temp. and diluted with water (50 mL). The resulting solution was extracted with EtOAc (100 mL). The organic layer was washed with a saturated NaCl solution (2 x 50 mL), dried (MgSO₄), and concentrated *in vacuo.* The residual solids were purified by MPLC (50% EtOAc/50% hexanes) to give the desired amine (0.650 g, 38%).

### A3k. General Method for Substituted Aniline Formation via Nitroarene Formation Through Nucleophilic Aromatic Substitution, Followed by Reduction

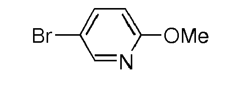

**Step 1. 5-Bromo-2-methoxypyridine**: A mixture of 2,5-dibromopyridine (5.5 g, 23.2 mmol) and NaOMe (3.76g, 69.6 mmol) in MeOH (60 mL) was heated at 70 °C in a sealed reaction vessel for 42 h, then allowed to cool to room temp. The reaction mixture was treated with water (50 mL) and extracted with EtOAc (2 x 100 mL). The combined organic layers were dried (Na₂SO₄) and concentrated under reduced pressure to give a pale yellow, volatile oil (4.1g, 95% yield): TLC (10% EtOAc / 90% hexane) R*_{f}* 0.57. 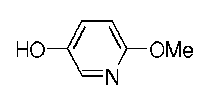 **Step 2. 5-Hydroxy-2-methoxypyridine**: To a stirred solution of 5-bromo-2-methoxypyridine (8.9 g, 47.9 mmol) in THF (175 mL) at -78 °C was added an n-butyllithium solution (2.5 M in hexane; 28.7 mL, 71.8 mmol) dropwise and the resulting mixture was allowed to stir at -78 °C for 45 min. Trimethyl borate (7.06 mL, 62.2 mmol) was added via syringe and the resulting mixture was stirred for an additional 2 h. The bright orange reaction mixture was warmed to 0 °C and was treated with a mixture of a 3 N NaOH solution (25 mL, 71.77 mmol) and a hydrogen peroxide solution (30%; approx. 50 mL). The resulting yellow and slightly turbid reaction mixture was warmed to room temp. for 30 min and then heated to the reflux temp. for 1 h. The reaction mixture was then allowed to cool to room temp. The aqueous layer was neutralized with a IN HCl solution then extracted with Et₂O (2 x 100 mL). The combined organic layers were dried (Na₂SO₄) and concentrated under reduced pressure to give a viscous yellow oil (3.5g, 60%).

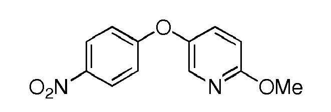

**Step 3. 4-(5-(2-Methoxy)pyridyl)oxy-1-nitrobenzene**: To a stirred slurry of NaH (97%, 1.0 g, 42 mmol) in anh DMF (100 mL) was added a solution of 5-hydroxy-2-methoxypyridine (3.5g, 28 mmol) in DMF (100 mL). The resulting mixture was allowed to stir at room temp. for 1 h, 4-fluoronitrobenzene (3 mL, 28 mmol) was added via syringe. The reaction mixture was heated to 95 °C overnight, then treated with water (25 mL) and extracted with EtOAc (2 x 75 mL). The organic layer was dried (MgSO₄) and concentrated under reduced pressure. The residual brown oil was crystalized EtOAc/hexane) to afford yellow crystals (5.23 g, 75%).

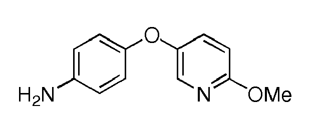

**Step 4. 4-(5-(2-Methoxy)pyridyl)oxyaniline**: 4-(5-(2-Methoxy)pyridyl)oxy-1-nitrobenzene was reduced to the aniline in a manner analogous to that described in Method A3d, Step2.

### A4a. General Method for Substituted Aniline Synthesis via Nucleophilic Aromatic Substitution using a Halopyridine

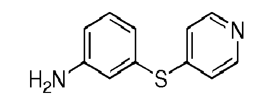

**3-(4-Pyridinylthio)aniline**: To a solution of 3-aminothiophenol (3.8 mL, 34 mmoles) in anh DMF (90mL) was added 4-chloropyridine hydrochloride (5.4 g, 35.6 mmoles) followed by K₂CO₃ (16.7 g, 121 mmoles). The reaction mixture was stirred at room temp. for 1.5 h, then diluted with EtOAc (100 mL) and water (100mL). The aqueous layer was back-extracted with EtOAc (2 x 100 mL). The combined organic layers were washed

with a saturated NaCl solution (100 mL), dried (MgSO₄), and concentrated under reduced pressure. The residue was filtered through a pad of silica (gradient from 50% EtOAc/50% hexane to 70% EtOAc/30% hexane) and the resulting material was triturated with a Et₂O/hexane solution to afford the desired product (4.6 g, 66%): TLC (100 % ethyl acetate) R*_{f}* 0.29; ¹H-NMR (DMSO-d₆) * 5.41 (s, 2H), 6.64-6.74 (m, 3H), 7.01 (d, J=4.8, 2H), 7.14 (t, J=7.8 Hz, 1H), 8.32 (d, J=4.8, 2H).

### A4b. General Method for Substituted Aniline Synthesis via Nucleophilic Aromatic Substitution using a Halopyridine

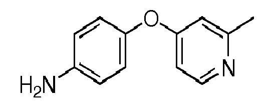

**4-(2-Methyl-4-pyridinyloxy)aniline**: To a solution of 4-aminophenol (3.6 g, 32.8 mmol) and 4-chloropicoline (5.0 g, 39.3 mmol) in anh DMPU (50 mL) was added potassium *tert-*butoxide (7.4 g, 65.6 mmol) in one portion. The reaction mixture was heated at 100 °C with stirring for 18 h, then was allowed to cool to room temp. The resulting mixture was poured into water (200 mL) and extracted with EtOAc (3 x 150 mL). The combined extracts were sequentially washed with water (3 x 100 mL) and a saturated NaCl solution (2 x 100 mL), dried (Na₂SO₄), and concentrated *in vacuo.* The resulting oil was purified by flash chromatography (50 % EtOAc/50% hexane) to afford the desired product as a yellow oil (0.7 g, 9%): CI-MS *m*/*z* 201 ((M+H)⁺).

### A4c. General Method for Substituted Aniline Synthesis via Nucleophilic Aromatic Substitution using a Halopyridine

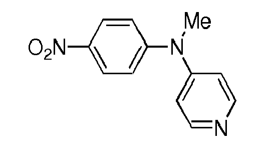

**Step 1. Methyl(4-nitrophenyl)-4-pyridylamine**: To a suspension of *N*-methyl-4-nitroaniline (2.0 g, 13.2 mmol) and K₂CO₃ (7.2 g, 52.2 mmol) in DMPU (30mL) was added 4-chloropyridine hydrochloride (2.36 g, 15.77 mmol). The reaction mixture was heated at 90 °C for 20 h, then cooled to room temperature. The resulting mixture was diluted with water (100 mL) and extracted with EtOAc (100 mL). The organic layer was washed with water (100 mL), dried (Na₂SO₄) and concentrated under reduced pressure. The residue was purified by column chromatography (silica gel, gradient from 80% EtOAc /20% hexanes to 100% EtOAc) to afford methyl(4-nitrophenyl)-4-pyridylamine (0.42 g)

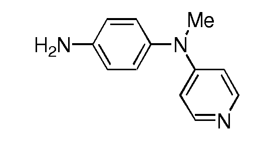

**Step 2. Methyl(4-aminophenyl)-4-pyridylamine**: Methyl(4-nitrophenyl)-4-pyridylamine was reduced in a manner analogous to that described in Method A1.

### A5. General Method of Substituted Aniline Synthesis via Phenol Alkylation Followed by Reduction of a Nitroarene

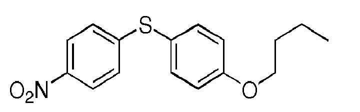

**Step 1. 4-(4-Butoxyphenyl)thio-1-nitrobenzene**: To a solution of 4-(4-nitrophenyl-thio)phenol (1.50 g, 6.07 mmol) in anh DMF (75 ml) at 0 °C was added NaH (60% in mineral oil, 0.267 g, 6.67 mmol). The brown suspension was stirred at 0 °C until gas evolution stopped (15 min), then a solution of iodobutane (1.12 g, .690 ml, 6.07 mmol) in anh DMF (20 mL) was added dropwise over 15 min at 0 °C. The reaction was stirred at room temp. for 18 h at which time TLC indicated the presence of unreacted phenol, and additional iodobutane (56 mg, 0.035 mL, 0.303 mmol, 0.05 equiv) and NaH (13 mg, 0.334 mmol) were added. The reaction was stirred an additional 6 h at room temp., then was quenched by the addition of water (400 mL). The resulting mixture was extracted with Et₂O (2 x 500 mL). The combined organics were washed with water (2 x 400 mL), dried (MgSO₄), and concentrated under reduced pressure to give a clear yellow oil, which was purified by silica gel chromatography (gradient from 20% EtOAc/80% hexane to 50% EtOAc/50% hexane) to give the product as a yellow solid (1.24 g, 67%): TLC (20% EtOAc/80% hexane) R*_{f}* 0.75; ¹H-NMR (DMSO-d₆) * 0.92 (t, *J*= 7.5 Hz, 3H), 1.42 (app hex, *J*=7.5 Hz, 2H), 1.70 (m, 2H), 4.01 (t, *J*= 6.6 Hz, 2H), 7.08 (d, *J*=8.7 Hz, 2H), 7.17 (d, *J*=9 Hz, 2H), 7.51 (d, *J*= 8.7 Hz, 2H), 8.09 (d, *J*= 9 Hz, 2H).

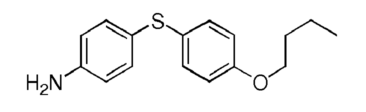

**Step 2. 4-(4-Butoxyphenyl)thioaniline**: 4-(4-Butoxyphenyl)thio-1-nitrobenzene was reduced to the aniline in a manner analagous to that used in the preparation of 3-(trifluoromethyl)-4-(4-pyridinylthio)aniline (Method A3b, Step 2): TLC (33% EtOAc/77% hexane) R*_{f}* 0.38.

### A6. General Method for Synthesis of Substituted Anilines by the Acylation of Diaminoarenes

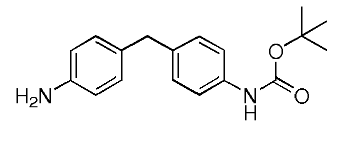

**4-(4-*tert*-Butoxycarbamoylbenzyl)aniline**: To a solution of 4,4'-methylenedianiline (3.00 g, 15.1 mmol) in anh THF (50 mL) at room temp was added a solution of di-*tert-*butyl dicarbonate (3.30 g, 15.1 mmol) in anh THF (10 mL). The reaction mixture was heated at the reflux temp. for 3 h, at which time TLC indicated the presence of unreacted methylenedianiline. Additional di-*tert*-butyl dicarbonate (0.664 g, 3.03 mmol, 0.02 equiv) was added and the reaction stirred at the reflux temp. for 16 h. The resulting mixture was diluted with Et₂O (200 mL), sequentially washed with a saturated NaHCO₃ solution (100 ml), water (100 mL) and a saturated NaCl solution (50 mL), dried (MgSO₄), and concentrated under reduced pressure. The resulting white solid was purified by silica gel chromatography (gradient from 33% EtOAc/67% hexane to 50% EtOAc/50% hexane) to afford the desired product as a white solid (2.09 g, 46%): TLC (50% EtOAc/50% hexane) R*_{f}* 0.45; ¹H-NMR (DMSO-d₆) * 1.43 (s, 9H), 3.63 (s, 2H), 4.85 (br s, 2H), 6.44 (d, *J*=8.4 Hz, 2H), 6.80 (d, *J*=8.1 Hz, 2H), 7.00 (d, *J*=8.4 Hz, 2H), 7.28 (d, *J*=8.1 Hz, 2H), 9.18 (br s, 1H); FAB-MS *m*/*z* 298 (M⁺).

### A7. General Method for the Synthesis of Aryl Amines via Electrophilic Nitration Followed by Reduction

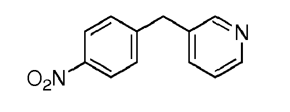

**Step 1. 3-(4-Nitrobenzyl)pyridine**: A solution of 3-benzylpyridine (4.0 g, 23.6 mmol) and 70% nitric acid (30 mL) was heated overnight at 50 °C. The resulting mixture was allowed to cool to room temp. then poured into ice water (350 mL). The aqueous mixture then made basic with a IN NaOH solution, then extracted with Et₂O (4 x 100 mL). The combined extracts were sequentially washed with water (3 x 100 mL) and a saturated NaCl solution (2 x 100 mL), dried (Na₂SO₄), and concentrated *in vacuo.* The residual oil was purified by MPLC (silica gel; 50 % EtOAc/50% hexane) then recrystallization (EtOAc/hexane) to afford the desired product (1.0 g, 22%): GC-MS *m*/*z* 214 (M⁺).

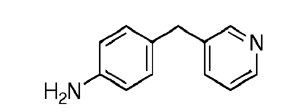

**Step 2. 3-(4-Pyridinyl)methylaniline**: 3-(4-Nitrobenzyl)pyridine was reduced to the aniline in a manner analogous to that described in Method A1.

### A8. General Method for Synthesis of Aryl Amines via Substitution with Nitrobenzyl Halides Followed by Reduction

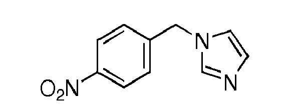

**Step 1. 4-(1-Imidazolylmethyl)-1-nitrobenzene**: To a solution of imidazole (0.5 g, 7.3 mmol) and 4-nitrobenzyl bromide (1.6 g, 7.3 mmol) in anh acetonitrile (30 mL) was added K₂CO₃ (1.0 g, 7.3 mmol). The resulting mixture was stirred at room temp. for 18 h and then poured into water (200 mL) and the resulting aqueous solution was extracted with EtOAc (3 x 50 mL). The combined organic layers were sequentially washed with water (3 x 50 mL) and a saturated NaCl solution (2 x 50 mL), dried (MgSO₄), and concentrated *in vacuo.* The residual oil was purified by MPLC (silica gel; 25% EtOAc/75% hexane) to afford the desired product (1.0 g, 91%): EI-MS *m*/*z* 203 (M⁺).

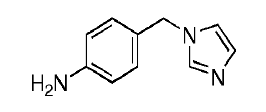

**Step 2. 4-(1-Imidazolylmethyl)aniline**: 4-(1-Imidazolylmethyl)-1-nitrobenzene was reduced to the aniline in a manner analogous to that described in Method A2.

### A9. Formation of Substituted Hydroxymethylanilines by Oxidation of Nitrobenzyl Compounds Followed by Reduction

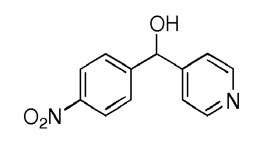

**Step 1. 4-(1-Hydroxy-1-(4-pyridyl)methyl-1-nitrobenzene**: To a stirred solution of 3-(4-nitrobenzyl)pyridine (6.0 g, 28 mmol) in CH₂Cl₂ (90 mL) was added *m*-CPBA (5.80 g, 33.6 mmol) at 10 °C, and the mixture was stirred at room temp. overnight. The reaction mixture was successively washed with a 10% NaHSO₃ solution (50 mL), a saturated K₂CO₃ solution (50 mL) and a saturated NaCl solution (50 mL), dried (MgSO₄) and concentrated under reduced pressure. The resulting yellow solid (2.68 g) was dissolved in anh acetic anhydride (30 mL) and heated at the reflux temperature overnight. The mixture was concentrated under reduced pressure. The residue was dissolved in MeOH (25 mL) and treated with a 20% aqueous NH₃ solution (30 mL). The mixture was stirred at room temp. for 1 h, then was concentrated under reduced pressure. The residue was poured into a mixture of water (50 mL) and CH₂Cl₂ (50 mL). The organic layer was dried (MgSO₄), concentrated under reduced pressure, and purified by column chromatography (80% EtOAc/ 20% hexane) to afford the desired product as a white solid. (0.53 g, 8%): mp 110-118 °C; TLC (80% EtOAc/20% hexane) R*_{f}* 0.12; FAB-MS *m*/*z* 367 ((M+H)⁺, 100%). 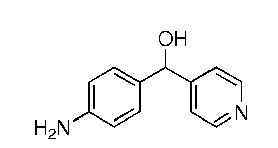 **Step 2. 4-(1-Hydroxy-1-(4-pyridyl)methylaniline**: 4-(1-Hydroxy-1-(4-pyridyl)-methyl-1-nitrobenzene was reduced to the aniline in a manner analogous to that described in Method A3d, Step2.

### A10. Formation of 2-(N-methylcarbamoyl)pyridines via the Menisci reaction

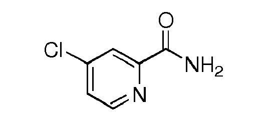

**Step 1. 2-(*N*-methylcarbamoyl)-4-chloropyridine**. (**Caution**: this is a highly hazardous, potentially explosive reaction.) To a solution of 4-chloropyridine (10.0 g) in *N-*methylformamide (250 mL) under argon at ambient temp was added conc. H₂SO₄ (3.55 mL) (exotherm). To this was added H₂O₂ (17 mL, 30% wt in H₂O) followed by FeSO_{4˙}7H₂O (0.55 g) to produce an exotherm. The reaction was stirred in the dark at ambient temp for 1h then was heated slowly over 4 h at 45 °C. When bubbling subsided,the reaction was heated at 60 °C for 16 h. The resulting opaque brown solution was diluted with H₂O (700 mL) followed by a 10% NaOH solution (250 mL). The resulting aqueous mixture was extracted with EtOAc (3 x 500 mL) and the organic layers were washed separately with a saturated NaCl solution (3 x 150 mL). The combined organics phases were dried (MgSO₄) and filtered through a pad of silica gel eluting with EtOAc. The solvent was removed in vacuo and the brown residue was purified by silica gel chromatography (gradient from 50% EtOAc / 50% hexane to 80% EtOAc / 20% hexane). The resulting yellow oil crystallized at 0 °C over 72 h to give 2-(N-methylcarbamoyl)-4-chloropyridine in yield (0.61 g, 5.3%): TLC (50% EtOAc/50% hexane) R*_{f}* 0.50; MS; ¹H NMR (CDCl₃): d 8.44 (d, 1 H, J = 5.1 Hz, CHN), 8.21 (s, 1H, CHCCO), 7.96 (b s, 1H, NH), 7.43 (dd, 1H, J = 2.4, 5.4 Hz, C1CHCN), 3.04 (d, 3H, J = 5.1 Hz, methyl); CI-MS *m*/*z* 171 ((M+H)+).

### Step 1b. Synthesis of 4-chloropyridine-2-carbonyl chloride HCl salt via picolinic acid

Anhydrous DMF (6.0 mL) was slowly added to SOCl₂ (180 mL) between 40° and 50 °C. The solution was stirred in that temperature range for 10 min. then picolinic acid (60.0 g, 487 mmol) was added in portions over 30 min. The resulting solution was heated at 72 °C (vigorous SO₂ evolution) for 16 h to generate a yellow solid precipitate. The resulting mixture was cooled to room temp., diluted with toluene (500 mL) and concentrated to 200 mL. The toluene addition/concentration process was repeated twice. The resulting nearly dry residue was filtered and the solids were washed with toluene (2 x 200 mL) and dried under high vacuum for 4 h to afford 4-chloropyridine-2-carbonyl chloride HCl salt as a yellow-orange solid (92.0 g, 89%).

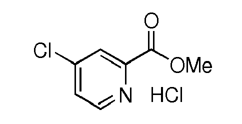

### Step 2. Synthesis of methyl 4-chloropyridine-2-carboxylate HCl salt

Anh DMF (10.0 mL) was slowly added to SOCl₂ (300 mL) at 40-48 °C. The solution was stirred at that temp. range for 10 min., then picolinic acid (100 g, 812 mmol) was added over 30 min. The resulting solution was heated at 72 °C (vigorous SO₂ evolution) for 16 h to generate a yellow solid. The resulting mixture was cooled to room temp., diluted with toluene (500 mL) and concentrated to 200 mL. The toluene addition/concentration process was repeated twice. The resulting nearly dry residue was filtered, and the solids were washed with toluene (50 mL) and dried under high vacuum for 4 hours to afford 4-chloropyridine-2-carbonyl chloride HCl salt as an off-white solid (27.2 g, 16%). This material was set aside.

The red filtrate was added to MeOH (200 mL) at a rate which kept the internal temperature below 55 °C. The contents were stirred at room temp. for 45 min., cooled to 5 °C and treated with Et₂O (200 mL) dropwise. The resulting solids were filtered, washed with Et₂O (200 mL) and dried under reduced pressure at 35 °C to provide methyl 4-chloropyridine-2-carboxylate HCl salt as a white solid (110 g, 65%): mp 108-112 °C; ¹H-NMR (DMSO-d₆) * 3.88 (s, 3H); 7.82 (dd, *J*=5.5, 2.2 Hz, 1H); 8.08 (d, *J*=2.2 Hz, 1H); 8.68 (d, *J*=5.5 Hz, 1H); 10.68 (br s, 1H); HPLC ES-MS *m*/*z* 172 ((M+H)⁺).

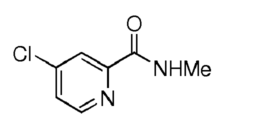

### Step 3a. Synthesis of 4-chloro-N-methyl-2-pyridinecarboxamide from methyl 4-chloropyridine-2-carboxylate

A suspension of methyl 4-chloropyridine-2-carboxylate HCl salt (89.0 g, 428 mmol) in MeOH (75 mL) at 0 °C was treated with a 2.0 M methylamine solution in THF (1 L) at a rate which kept the internal temp. below 5 °C. The resulting mixture was stored at 3 °C for 5 h, then concentrated under reduced pressure. The resulting solids were suspended in EtOAc (1 L) and filtered. The filtrate was washed with a saturated NaCl solution (500 mL), dried (Na₂SO₄) and concentrated under reduced pressure to afford 4-chloro-N-methyl-2-pyridinecarboxamide as pale-yellow crystals (71.2 g, 97%): mp 41-43 °C; ¹H-NMR (DMSO-d₆) * 2.81 (s, 3H), 7.74 (dd, *J*=5.1, 2.2 Hz, 1H), 8.00 (d, *J*=2.2, 1H), 8.61 (d, *J*=5.1 Hz, 1H), 8.85 (br d, 1H); CI-MS *m*/*z* 171 ((M+H)⁺).

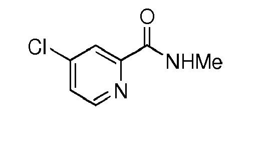

### Step 3b. Synthesis of 4-chloro-N-methyl-2-pyridinecarboxamide from 4-chloropyridine-2-carbonyl chloride

4-Chloropyridine-2-carbonyl chloride HCl salt (7.0 g, 32.95 mmol) was added in portions to a mixture of a 2.0 M methylamine solution in THF (100 mL) and MeOH (20 mL) at 0 °C. The resulting mixture was stored at 3 °C for 4 h, then concentrated under reduced pressure. The resulting nearly dry solids were suspended in EtOAc (100 mL) and filtered. The filtrate was washed with a saturated NaCl solution (2 x 100 mL), dried (Na₂SO₄) and concentrated under reduced pressure to provide 4-chloro-*N*-methyl-2-pyridinecarboxamide as a yellow, crystalline solid (4.95 g, 88%): mp 37-40 °C.

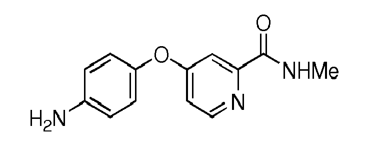

### Step 4. Synthesis of 4-(2-(N-methylcarbamoyl)-4-pyridyloxy)aniline

A solution of 4-aminophenol (9.60 g, 88.0 mmol) in anh. DMF (150 mL) was treated with potassium *tert*-butoxide (10.29 g, 91.7 mmol), and the reddish-brown mixture was stirred at room temp. for 2 h. The contents were treated with 4-chloro-*N*-methyl-2-pyridinecarboxamide (15.0 g, 87.9 mmol) and K₂CO₃ (6.50 g, 47.0 mmol) and then heated at 80 °C for 8 h. The mixture was cooled to room temp. and separated between EtOAc (500 mL) and a saturated NaCl solution (500 mL). The aqueous phase was back-extracted with EtOAc (300 mL). The combined organic layers were washed with a saturated NaCl solution (4 x 1000 mL), dried (Na₂SO₄) and concentrated under reduced pressure. The resulting solids were dried under reduced pressure at 35 °C for 3 h to afford 4-(2-(*N*-methylcarbamoyl)-4-pyridyloxy)aniline as a light-brown solid 17.9 g, 84%): ¹H-NMR (DMSO-d₆) * 2.77 (d, *J*=4.8 Hz, 3H), 5.17 (br s, 2H), 6.64, 6.86 (AA'BB' quartet, *J*=8.4 Hz, 4H), 7.06 (dd, *J*=5.5, 2.5 Hz, 1H), 7.33 (d, *J*=2.5 Hz, 1H), 8.44 (d, *J*=5.5 Hz, 1H), 8.73 (br d, 1H); HPLC ES-MS *m*/*z* 244 ((M+H)⁺).

### A11. General Method for the Synthesis of 5-(4-Aminophenoxy)isoindoline-1,3-dione

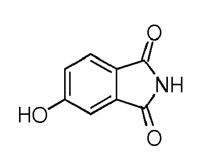

### Step 1. Synthesis of 5-hydroxyisoindoline-1,3-dione

To a mixture of ammonium carbonate (5.28 g, 54.9 mmol) in conc. AcOH (25 mL) was slowly added 4-hydroxyphthalic acid (5.0 g, 27.45 mmol). The resulting mixture was heated at 120 °C for 45 min., then the clear, bright yellow mixture was heated at 160 °C for 2 h. The resulting mixture was maintained at 160 °C and was concentrated to approximately 15 mL, then was cooled to room temp. and adjusted pH 10 with a IN NaOH solution. This mixture was cooled to 0 °C and slowly acidified to pH 5 using a IN HCl solution. The resultant precipitate was collected by filtration and dried under reduced pressure to yield 5-hydroxyisoindoline-1,3-dione as a pale yellow powder as product (3.24 g, 72%): ¹H NMR (DMSO-d₆) * 7.00-7.03 (m, 2H), 7.56 (d, *J*=9.3Hz, 1H).

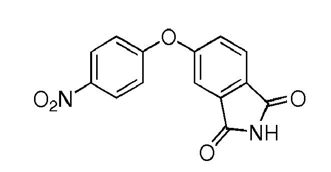

### Step 2. Synthesis of 5-(4-nitrophenoxy)isoindoline-1,3-dione

To a stirring slurry of NaH (1.1 g, 44.9 mmol) in DMF (40 mL) at 0 °C was added a solution of 5-hydroxyisoindoline-1,3-dione (3.2 g, 19.6 mmol) in DMF (40 mL) dropwise. The bright yellow-green mixture was allowed to return to room temp. and was stirred for 1 h, then 1-fluoro-4-nitrobenzene (2.67 g, 18.7 mmol) was added via syringe in 3-4 portions. The resulting mixture was heated at 70 °C overnight, then cooled to room temp. and diluted slowly with water (150 mL), and extracted with EtOAc (2 x 100 mL). The combined organic layers were dried (MgSO₄) and concentrated under reduced pressure to give 5-(4-nitrophenoxy)isoindoline-1,3-dione as a yellow solid (3.3 g, 62%): TLC (30% EtOAc/70% hexane) R*_{f}* 0.28; 1H NMR (DMSO-d₆) * 7.32 (d, *J*=12 Hz, 2H), 7.52-7.57 (m, 2H), 7.89(d, *J*=7.8 Hz, 1H), 8.29 (d, *J*=9 Hz, 2H), 11.43 (br s, 1H); CI-MS *m*/*z* 285 ((M+H)⁺, 100%).

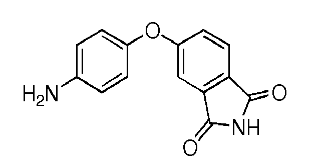

### Step 3. Synthesis of 5-(4-aminophenoxy)isoindoline-1,3-dione

A solution of 5-(4-nitrophenoxy)isoindoline-1,3-dione (0.6 g, 2.11 mmol) in conc. AcOH (12 mL) and water (0.1 mL) was stirred under stream of argon while iron powder (0.59 g, 55.9 mmol) was added slowly. This mixture stirred at room temp. for 72 h, then was diluted with water (25 mL) and extracted with EtOAc (3 x 50 mL). The combined organic layers were dried (MgSO₄) and concentrated under reduced pressure to give 5-(4-aminophenoxy)isoindoline-1,3-dione as a brownish solid (0.4 g, 75%): TLC (50% EtOAc/50% hexane) R*_{f}* 0.27; ¹H NMR (DMSO-d₆) * 5.14 (br s, 2H), 6.62 (d, *J*=8.7 Hz, 2H), 6.84 (d, *J*=8.7 Hz, 2H), 7.03 (d, *J*=2.1 Hz, 1H), 7.23 (dd, 1H), 7.75 (d, *J*=8.4 Hz, 1H), 11.02 (s, 1H); HPLC ES-MS *m*/*z* 255 ((M+H)⁺, 100%).

### A12. General Method for the Synthesis of *-Sulfonylphenyl Anilines

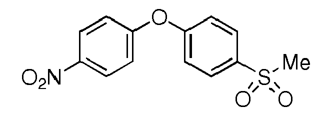

**Step 1. 4-(4-Methylsulfonylphenoxy)-1-nitrobenzene: To a solution of 4-(4-**methylthiophenoxy)-1-ntirobenzene (2 g, 7.66 mmol) in CH₂Cl₂ (75 mL) at 0 °C was slowly added *m*CPBA (57-86%, 4 g), and the reaction mixture was stirred at room temperature for 5 h. The reaction mixture was treated with a 1 N NaOH solution (25 mL). The organic layer was sequentially washed with a IN NaOH solution (25 mL), water (25 mL) and a saturated NaCl solution (25 mL), dried (MgSO₄), and concentrated under reduced pressure to give 4-(4-methylsulfonylphenoxy)-1-nitrobenzene as a solid (2.1 g).

**Step 2. 4-(4-Methylsulfonylphenoxy)-1-aniline:** 4-(4-Methylsulfonylphenoxy)-1-nitrobenzene was reduced to the aniline in a manner anaologous to that described in Method A3d, step 2.

### A13. General Method for Synthesis of *-Alkoxy-*-carboxyphenyl Anilines

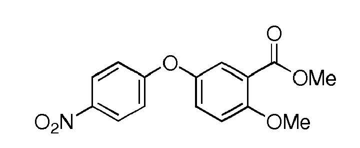

**Step 1. 4-(3-Methoxycarbonyl-4-methoxyphenoxy)-1-nitrobenzene**: To a solution of -(3-carboxy-4-hydroxyphenoxy)-1-nitrobenzene (prepared in a manner analogous to that described in Method A3a, step 1, 12 mmol) in acetone (50 mL) was added K₂CO₃ (5 g) and dimethyl sulfate (3.5 mL). The resulting mixture was heated at the reflux temperature overnight, then cooled to room temperature and filtered through a pad of Celite^{®}. The resulting solution was concentrated under reduced pressure, absorbed onto silica gel, and purified by column chromatography (50% EtOAc / 50% hexane) to give 4-(3-methoxycarbonyl-4-methoxyphenoxy)-1-nitrobenzene as a yellow powder (3 g): mp 115-118 °C.

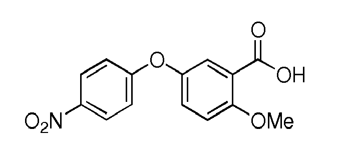

**Step 2. 4-(3-Carboxy-4-methoxyphenoxy)-1-nitrobenzene**: A mixture of 4-(3-methoxycarbonyl-4-methoxyphenoxy)-1-nitrobenzene (1.2 g), KOH (0.33 g),and water (5 mL) in MeOH (45 mL) was stirred at room temperature overnight and then heated at the reflux temperature for 4 h. The resulting mixture was cooled to room temperature and concentrated under reduced pressure. The residue was dissolved in water (50 mL), and the aqueous mixture was made acidic with a IN HCl solution. The resulting mixture was extracted with EtOAc (50 mL). The organic layer was dried (MgSO₄) and concentrated under reduced pressure to give 4-(3-carboxy-4-methoxyphenoxy)-1-nitrobenzene (1.04 g).

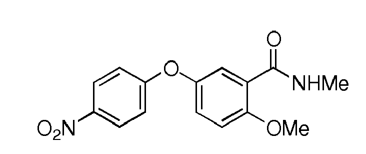

### Step 3. 4-(3-(N-Methylcarbamoly)-4-methoxyphenoxy)-1-nitrobenzene:

To a solution of 4-(3-carboxy-4-methoxyphenoxy)-1-nitrobenzene (0.50 g, 1.75 mmol) in CH₂Cl₂ (12 mL) was added SOCl₂ (0.64 mL, 8.77 mmol) in portions. The resulting solution was heated at the reflux temp. for 18 h, cooled to room temp., and concentrated under reduced pressure. The resulting yellow solids were dissolved in CH₂Cl₂ (3 mL) then the resulting solution was treated with a methylamine solution (2.0 M in THF, 3.5 mL, 7.02 mmol) in portions (CAUTION: gas evolution), and stirred at room temp. for 4 h. The resulting mixture was treated with a IN NaOH solution, then extracted with CH₂Cl₂ (25 mL). The organic layer was dried (Na₂SO₄) and concentrated under reduced pressure to give 4-(3-(*N*-methylcarbamoly)-4-methoxyphenoxy)-1-nitrobenzene as a yellow solid (0.50 g, 95%).

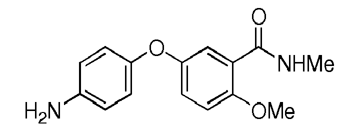

### Step 4. 4-(3-(N-Methylcarbamoly)-4-methoxyphenoxy)aniline:

A slurry of 4-(3-(*N*-methylcarbamoly)-4-methoxyphenoxy)-1-nitrobenzene (0.78 g, 2.60 mmol) and 10% Pd/C (0.20 g) in EtOH (55 mL) was stirred under 1 atm of H₂ (balloon) for 2.5 d, then was filtered through a pad of Celite^{®}. The resulting solution was concentrated under reduced pressure to afford 4-(3-(*N*-methylcarbamoly)-4-methoxyphenoxy)aniline as an off-white solid (0.68 g, 96%): TLC (0.1% Et₃N/99.9% EtOAc) R*_{f}* 0.36.

### A14. General Method for the Synthesis of 4-(3-N-Methylcarbamoylphenoxy)aniline.

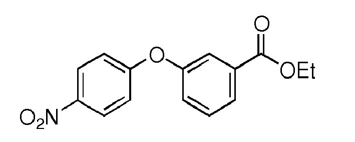

### Step 1. Synthesis of 4-(3-ethoxycarbonylphenoxy)-1-nitrobenzene

A mixture of 4-fluoro-1-nitrobenzene (16 mL, 150 mmol), ethyl 3-hydroxybenzoate 25 g, 150 mmol) and K₂CO₃ (41 g, 300 mmol) in DMF (125 mL) was heated at the reflux temp. overnight, cooled to room temp. and treated with water (250 mL). The resulting mixture was extracted with EtOAc (3 x 150 mL). The combined organic phases were sequentially washed with water (3 x 100 mL) and a saturated NaCl solution (2 x 100 mL), dried (Na₂SO₄) and concentrated under reduced pressure. The residue was purified by column chromatography (10% EtOAc/90% hexane) to afford 4-(3-ethoxycarbonylphenoxy)-1-nitrobenzene as an oil (38 g).

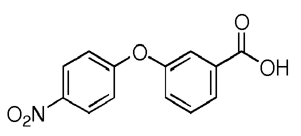

### Step 2. Synthesis of 4-(3-carboxyphenoxy)-1-nitrobenzene

To a vigorously stirred mixture of 4-(3-ethoxycarbonylphenoxy)-1-nitrobenzene (5.14 g, 17.9 mmol) in a 3:1 THF/water solution (75 mL) was added a solution LiOH•H₂O (1.50 g, 35.8 mmol) in water (36 mL). The resulting mixture was heated at 50 °C overnight, then cooled to room temp., concentrated under reduced pressure, and adjusted to pH 2 with a 1M HCl solution. The resulting bright yellow solids were removed by filtration and washed with hexane to give 4-(3-carboxyphenoxy)-1-nitrobenzene (4.40 g, 95%).

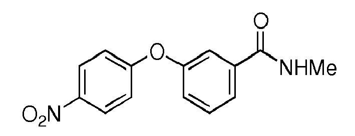

### Step 3. Synthesis of 4-(3-(N-methylcarbamoyl)phenoxy)-1-nitrobenzene

A mixture of 4-(3-carboxyphenoxy)-1-nitrobenzene (3.72 g, 14.4 mmol), EDCl•HCl (3.63 g, 18.6 mmol), *N*-methylmorpholine (1.6 mL, 14.5 mmol) and methylamine (2.0 M in THF; 8 mL, 16 mmol) in CH₂Cl₂ (45 mL) was stirred at room temp. for 3 d, then concentrated under reduced pressure. The residue was dissolved in EtOAc (50 mL) and the resulting mixture was extracted with a 1M HCl solution (50 mL). The aqueous layer was back-extracted with EtOAc (2 x 50 mL). The combined organic phases were washed with a saturated NaCl solution (50 mL), dried (Na₂SO₄), and concentrated under reduced pressure to give 4-(3-(*N*-methylcarbamoyl)phenoxy)-1-nitrobenzene as an oil (1.89 g).

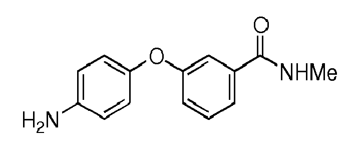

### Step 4. Synthesis of 4-(3-(N-methylcarbamoyl)phenoxy)aniline

A slurry of 4-(3-(*N*-methylcarbamoyl)phenoxy)-1-nitrobenzene (1.89 g, 6.95 mmol) and 5% Pd/C (0.24 g) in EtOAc (20 mL) was stirred under an H₂ atm (balloon) overnight. The resulting mixture was filtered through a pad of Celite^{®} and concentrated under reduced pressure. The residue was purified by column chromatography (5% MeOH/95% CH₂Cl₂). The resulting oil solidified under vacuum overnight to give 4-(3-(*N-*methylcarbamoyl)phenoxy)aniline as a yellow solid (0.95 g, 56%).

### B. General Methods of Urea Formation

### B1. Reaction of a Heterocyclic Amine with an Aryl Isocyanate

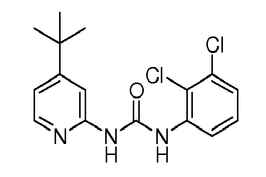

***N*-(4-*tert*-butylpyridyl)-*N*'-(2,3-dichlorophenyl) urea**: A solution of 2-amino-4-*tert-*butylpyridine (192 mg) and 2,3-dichlorophenyl isocyanate (240 mg) in anh. toluene (15 mL) was heated at 70 °C under argon for 24 h. The resulting mixture was diluted with EtOAc (200 mL) then washed with water (125 mL). The organic layer was dried (MgSO₄) and concentrated under reduced pressure to give a gum. Trituration of the gum with hexanes afforded *N*-(4-*tert*-butylpyridyl)-*N*'-(2,3-dichlorophenyl) urea as a white solid (394 mg, 91%): TLC (2:1 hexanes/ethyl acetate) R*_{f}* 0.40; FAB-MS *m*/*z* 338 ((M+H)⁺).

### B2a. Reaction of a Heterocyclic Amine with N,N'-Carbonyldiimidazole Followed by Reaction with a Substituted Aniline

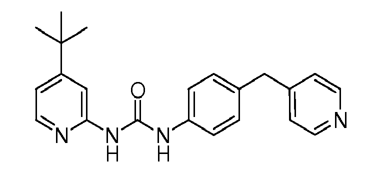

***N*-(4-*tert*-butylpyridyl)-*N*'-(4-(4-pyridinylmethyl)phenyl urea**: To a stirring solution of 4-*tert*-butyl-2-aminopyridine (192 mg) in anh. CH₂Cl₂ (15 mL) under argon at 0 °C was added CDI (207 mg). The resulting solution was allowed to warm to ambient temp over 2 h. To this mixture was added 4-(4-pyridylmethyl)aniline (prepared according to Method A1, 235 mg). The resulting solution was stirred at room temperature for 24 h, then was quenched with water (125 mL). The resulting mixture was extracted with EtOAc (200 mL). The organic layer was washed with water (100 mL), dried (MgSO₄) and concentrated under reduced pressure. The residue was purified by chromatography (SiO₂, EtOAc) to afford *N*-(4-*tert*-butylpyridyl)-*N*'-(4-(4-pyridinylmethyl)phenyl urea as a white solid (200 mg, 43%): TLC (EtOAc) R*_{f}* 0.47; FAB-MS *m*/*z* 361 ((M+H)⁺).

### B2b. Reaction of a Heterocyclic Amine with N,N'-Carbonyldiimidazole Followed by Reaction with a Substituted Aniline

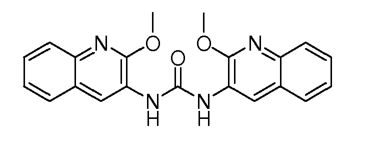

***N,N*'-(Bis(3-(2-methoxyquinolinyl)) urea)**: To a stirring solution of 3-amino-2-methoxyquinoline (138 mg) in anh CH₂Cl₂ (15 mL) under argon at 0 °C was added CDI (128 mg). The resulting solution was warmed to ambient temp over 1 h. After 16 h 4-(2-*N*-Methylcarbamyl-4-pyridyloxy)aniline (175 mg) was added and the resulting yellow solution was stirred at room temperature under argon for 72 h. The solution was treated with water (125 mL) and the resulting mixture was extracted with EtOAc (2 x 150 mL). The combined organics were washed with a saturated NaCl solution (100 mL), dried (MgSO₄) and concentrated under reduced pressure. The residue was triturated with a 10% hexane/90% EtOAc solution. The resulting white crystals were washed with EtOAc. The resulting filtrate was purified by chromatography (SiO₂, 50% EtOAc/50% hexane) to give *N,N*'-(bis(3-(2-methoxyquinolinyl)) urea) (30 mg, 20% yield): TLC (50% EtOAc/50% hexane) R*_{f}* 0.45; HPLC ES-MS *m*/*z* 375 ((M+H)⁺).

### B2c. Reaction of a Heterocyclic Amine with N,N'-Carbonyldiimidazole Followed by Reaction with a Substituted Aniline

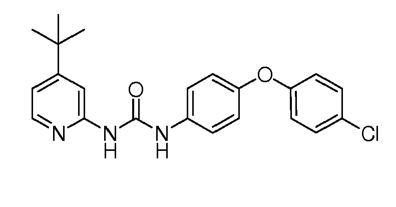

***N*-(4-*tert*-Butylpyridyl)-*N*'-(4-(4-chlorophenoxy)phenyl) urea**: A solution of 4-*tert-*butyl-2-aminopyridine (0.177 g, 1.18 mmol, 1 equiv.) in 1.2 mL of anh. CH₂Cl₂ (1.2 mL) was added to CDI (0.200 g, 1.24 mmol, 1.05 equiv) and the mixture was allowed to stir under argon at room temperature 1 d. To the resulting solution was added 4-(4-chlorophenoxy)aniline (0.259 g, 1.18 mmol, 1 equiv.) in one portion. The resulting mixture was stirred at room temperature for 1 d, then was treated with a 10% citric acid solution (2 mL) and allowed to stir for 1 h. The resulting organic layer was extracted with EtOAc (3 x 5 mL). The combined organic layers were dried (MgSO₄) and concentrated *in vacuo.* The resultant residue was treated with CH₂Cl₂ (10 mL) and a 1 N aqueous NaOH solution. This mixture was allowed to stir overnight. The resulting organic layer was extracted with CH₂Cl₂ (3 x 5 mL). The combined organic layers were (MgSO₄) and concentrated *in vacuo.* The resultant solids were suspended in diethyl ether (10 mL) and sonicated for 15 minutes. The resulting white solids were dried to give *N*-(4-*tert-*butylpyridyl)-*N*'-(4-(4-chlorophenoxy)phenyl) urea (42 mg, 9%): mp 198-199 °C.

### B3. Reaction of Substituted Aniline with N,N'-Carbonyldiimidazole Followed by Reaction with a Heterocyclic Amine

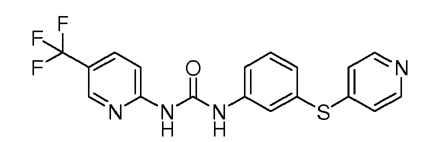

***N*-(2-(5-trifluoromethyl)pyridyloxy)-*N'*-(3-(4-pyridylthio)phenyl) urea**: A solution of 3-(4-pyridylthio)aniline (300 mg, 1.48 mmoles) in CH₂Cl₂ (12 mL) was treated with CDI (253 mg, 1.56 mmoles). The solution was stirred at room temperature and under argon for 2 h. The resulting mixture was treated with 2-amino-5-(trifluoromethyl)pyridine (238 mg, 1.47 mmoles) and heated at 40 °C overnight. The reaction mixture was then diluted with EtOAc (25 mL), washed with water (10 mL) and a saturated NaCl solution m(25 mL), dried (MgSO₄), and concentrated under reduced pressure. The residue was purified by column chromatography (SiO₂; gradient from 70% EtOAc/30% CH₂Cl₂ to 100% EtOAc to give *N*-(2-(5-trifluoromethyl)pyridyloxy)-*N*'-(3-(4-pyridylthio)phenyl) urea afforded (103 mg): TLC (50% EtOAc/50% CH₂Cl₂) R*_{f}* 0.33; ¹H-NMR (DMSO-d₆) 6.06 (d, *J*=6Hz, 2H), 7.25 (dt, *J*=1.2,7.8 Hz, 1H), 7.48 (t, *J*=8.1 Hz, 1H), 7.59-7.63 (m, 1H), 7.77 (d, *J*=8.7 Hz, 1H), 7.86 (t, *J*=1.8 Hz, 1H), 8.12 (dd, *J*=2.7,9.3 Hz, 1H), 8.37 (d, *J*=6.3 Hz, 2H), 8.67 (bs, 1H), 9.88 (s, 1H), 10.26 (s, 1 H); FAB-MS *m*/*z* 391 ((M+H)⁺).

### B4. Reaction of a Heterocyclic Amine with Phosgene, Followed by Reaction with a Substituted Aniline

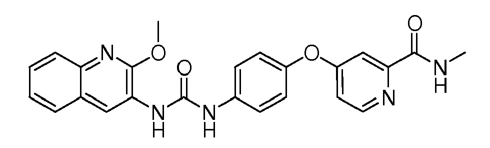

***N*-(3-(2-methoxyquinolinyl)-*N*'-(4-(4-(2-*N*-Methylcarbamyl-4-pyridyloxy)phenyl)**

**urea**: To a stirring solution of phosgene (20% in toluene, 1.38 mL) in anh. CH₂Cl₂ (20 ml) at 0 °C under argon was added anh. pyridine (207 mg) followed by 3-amino-2-methoxyquinoline (456 mg). The resulting solution was warmed to ambient temperature over 1 h, then concentrated in vacuo at ambient temperature to give a white solid. The solid was dried under vacuum for 15 min then suspended in anh toluene (20 mL). To the resulting slurry was added 4-(4-(2-(methylcarbamoyl)pyridyloxy)aniline (prepared according to Method A2, 300 mg) and the reaction heated under argon at 80 °C for 20 h. The resulting mixture was diluted with water (200 mL), then treated with a saturated NaHCO₃ solution (10 mL) and extracted with EtOAc (2 x 300 mL). The combined organic layers were washed with a saturated NaCl solution (100 mL), dried (MgSO4) and concentrated under reduced pressure. The solid yellow residue was purified by chromatography (SiO₂, gradient from 50% EtOAc/50% hexane to 100% EtOAc), followed by recrystallization from diethyl ether and hexane to give *N*-(3-(2-methoxyquinolinyl)-*N*'-(4-(4-(2-*N*-Methylcarbamyl-4-pyridyloxy)phenyl) urea as a white solid (140 mg, 25%): TLC (EtOAc) R*_{f}* 0.52; FAB-MS *m*/*z* 430 ((M+H)⁺).

### B5a. Reaction of an Aniline with N,N'-Carbonyl Diimidazole Followed by Addition of a Second Aniline.

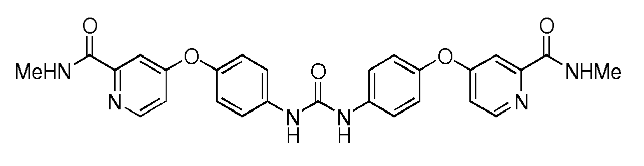

### Bis(4-(2-(N-methylcarbamoyl)-4-pyridyloxy)phenyl) Urea

To a stirring solution of 3-amino-2-methoxyquinoline (0.14 g) in anhydrous CH₂Cl₂ (15 mL) at 0 C was added CDI (0.13 g). The resulting solution was allowed to warm to room temp. over 1 h then was stirred at room temp. for 16 h. The resulting mixture was treated with 4-(2-(*N*-methylcarbamoyl)-4-pyridyloxy)aniline (0.18 g). The resulting yellow solution stirred at room temp. for 72 h, then was treated with water (125 mL). The resulting aqueous mixture was extracted with EtOAc (2 x 150 mL). The combined organic phases were washed with a saturated NaCl solution (100 ml), dried (MgSO₄) and concentrated under reduced pressure. The residue was triturated (90% EtOAc/10% hexane). The resulting white solids were collected by filtration and washed with EtOAc to give bis(4-(2-(*N*-methylcarbamoyl)-4-pyridyloxy)phenyl) urea (0.081 g, 44%): TLC (100% EtOAc) R*_{f}* 0.50; ¹H NMR (DMSO-d₆) 2.76 (d, *J*=5.1 Hz, 6H), 7.1-7.6 (m, 12H), 8.48 (d, *J*=5.4 Hz, 1H), 8.75 (d, *J*=4.8 Hz, 2H), 8.86 (s, 2H); HPLC ES-MS *m*/*z* 513 ((M+H)⁺).

### B5b. Reaction of an Isocyanate with an Aniline.

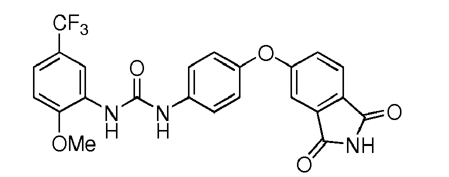

### N-(2-Methoxy-5-(trifluoromethyl)phenyl-N'-(4-(1,3-dioxoisoindolin-5-yloxy)phenyl) Urea

To a stirring solution of 2-methoxy-5-(trifluoromethyl)phenyl isocyanate (0.10 g, 0.47 mmol) in CH₂Cl₂ (1.5 mL) was added 5-(4-aminophenoxy)isoindoline-1,3-dione (Method A3, Step 3; 0.12 g, 0.47 mmol) in one portion. The resulting mixture was stirred for 12 h, then was treated with CH₂Cl₂ (10 mL) and MeOH (5 mL). The resulting mixture was sequentially washed with a 1N HCl solution (15 mL) and a saturated NaCl solution (15 mL), dried (MgSO₄) and concentrated under reduced pressure to afford *N*-(2-methoxy-5-(trifluoromethyl)phenyl-*N*'-(4-(1,3-dioxoisoindolin-5-yloxy)phenyl) urea as a white solid (0.2 g, 96%): TLC (70% EtOAc/30% hexane) R*_{f}* 0.50; ¹H NMR (DMSO-d₆) 3.95 (s, 3H), 7.31-7.10 (m, 6H), 7.57 (d, J=9.3Hz, 2H), 7.80 (d, J=8.7 Hz, 1H), 8.53 (br s, 2H), 9.57 (s, 1H), 11.27 (br s, 1H); HPLC ES-MS 472.0 ((M+H)⁺, 100%).

### B6. Reaction of an Aniline with Phosgene Followed by Addition of a Second Aniline.

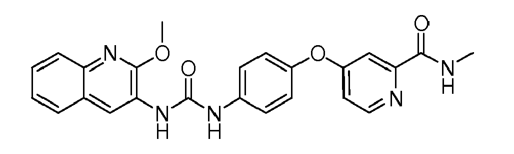

### N-(3-(2-methoxyquinolinyl)-N'-(4-(4-(2-N-Methylcarbamyl-4-pyridyloxy)phenyl) Urea

To a stirring solution of phosgene (20% in toluene, 1.38 mL) in anh. CH₂Cl₂ (20 ml) at 0 °C under argon was added anh. pyridine (207 mg) followed by 3-amino-2-methoxyquinoline (456 mg). The resulting solution was warmed to ambient temperature over 1 h, then concentrated in vacuo at ambient temperature to give a white solid. The solid was dried under vacuum for 15 min then suspended in anh toluene (20 mL). To the resulting slurry was added 4-(4-(2-(methylcarbamoyl)pyridyloxy)aniline (prepared according to Method A2, 300 mg) and the reaction heated under argon at 80 °C for 20 h. The resulting mixture was diluted with water (200 mL), then treated with a saturated NaHCO₃ solution (10 mL) and extracted with EtOAc (2 x 300 mL). The combined organic layers were washed with a saturated NaCl solution (100 mL), dried (MgSO4) and concentrated under reduced pressure. The solid yellow residue was purified by chromatography (SiO₂, gradient from 50% EtOAc/50% hexane to 100% EtOAc), followed by recrystallization from diethyl ether and hexane to give *N*-(3-(2-methoxyquinolinyl)-*N*'-(4-(4-(2-*N*-Methylcarbamyl-4-pyridyloxy)phenyl) urea as a white solid (140 mg, 25%): TLC (EtOAc) R_{f} 0.52; FAB-MS *m*/*z* 430 ((M+H)⁺).

### SPECIFIC COMPOUND PREPARATIONS

Descriptions of the detailed preparative steps used to prepare the specific compounds listed in Tables 1-8 are provided below. Many of the compounds listed in the Tables can be synthesized following a variety of methods. The specific examples below are therefore provided by way of illustration only and should not be construed to limit the scope of the invention in any way.

Entry 1: 4-*tert*-Butyl-2-aminopyridine was reacted with 4-tolyl isocyanate according to Method B 1 to afford the urea.

Entry 2: 4-*tert*-Butyl-2-aminopyridine was reacted with 4-fluorophenyl isocyanate according to Method B 1 to afford the urea.

Entry 3: *N*-(4-*tert*-Butylpyridinyl)-*N'*-(2,3-dichlorophenyl) urea was prepared according to Method B1.

Entry 4: 4-*tert*-Butyl-2-aminopyridine was reacted with 1-naphthyl isocyanate according to Method B 1 to afford the urea.

Entry 5: *N*-(4-*tert*-Butylpyridyl)-*N'*-(4-(4-pyridinylmethyl)phenyl urea was prepared according to Method B2a.

Entry 6: 4-*tert*-Butyl-2-aminopyridine was reacted with 4-phenoxyaniline according to Method B2c to afford the urea.

Entry 7: 4-*tert*-Butyl-2-aminopyridine was reacted with 4-(4-methylphenoxy)aniline according to Method B2c to afford the urea.

Entry 8: *N*-(4-*tert*-Butylpyridyl)-*N'*-(4-(4-chlorophenoxy)phenyl) urea was prepared according to Method B2c.

Entry 9: 4-*tert*-Butyl-2-aminopyridine was reacted with 4-(4-methoxyphenoxy)aniline according to Method B2c to afford the urea.

Entry 10: 4-(4-Aminophenoxy)pyridine was prepared starting from 4-hydroxypyridine and 1-bromo-3-nitrobenzene according to Method A3F. 4-*tert*-Butyl-2-aminopyridine was reacted with 4-(4-aminophenoxy)pyridine according to Method B2a to afford the urea.

Entry 11: 4-(4-Pyridylthio)aniline was prepared starting from 4-aminothiophenol and 4-chloropyridine hydrochloride according to Method A4a. 4-*tert*-Butyl-2-aminopyridine was reacted with 4-(4-pyridylthio)aniline according to Method B2c to afford the urea.

Entry 12: 4-(4-Pyridylthio)aniline was prepared starting from 4-aminothiophenol and 4-chloropyridine hydrochloride according to Method A4a. 4-*tert*-Butyl-2-aminopyridine was reacted with 3-(4-pyridylthio)aniline according to Method B2c to afford the urea.

Entry 13: 2-Amino-5-(trifluoromethyl)pyridine and 4-(4-pyridylmethyl)aniline were reacted according to Method B3 to afford the urea.

Entry 14: *N*-(2-(5-Trifluoromethyl)pyridyloxy)-*N'*-(3-(4-pyridylthio)phenyl) urea was prepared according to Method B3.

Entry 15: 3-Aminoisoquinoline was reacted with 4-tolyl isocyanate according to Method B 1 to afford the urea.

Entry 16: 3-Aminoisoquinoline was reacted with 4-fluorophenyl isocyanate according to Method B 1 to afford the urea.

Entry 17: 3-Aminoisoquinoline was reacted with 2,3-dichlorophenyl isocyanate according to Method B 1 to afford the urea.

Entry 18: 3-Aminoisoquinoline was reacted with 1-naphthyl isocyanate according to Method B 1 to afford the urea.

Entry 19: 3-Aminoisoquinoline was reacted with 4-(4-pyridylmethyl)aniline according to Method B2a to afford the urea.

Entry 20: 4-(4-Aminophenoxy)pyridine was prepared starting from 4-hydroxypyridine and 1-bromo-3-nitrobenzene according to Method A3f. 3-Aminoisoquinoline was reacted with 4-(4-aminophenoxy)pyridine according to Method B2a to afford the urea.

Entry 21: 3-Aminoquinoline and 4-(4-pyridylmethyl)aniline were reacted according to Method B3 to afford the urea.

Entry 22: *N,N'*-(Bis(3-(2-methoxyquinolinyl)) urea) was prepared according to Method B2b.

Entry 23: 3-Amino-2-methoxyquinoline and 4-(4-pyridylmethyl)aniline were reacted according to Method B3 to afford the urea.

Entry 24: 3-Amino-2-methoxyquinoline was reacted with 4-(4-pyridylcarbonyl)aniline according to Method B4 to afford the urea.

Entry 25: 4-(4-Pyridyloxy)aniline was prepared starting from 4-hydroxypyridine and 1-fluoro-4-nitrobenzene according to Method A3d. 3-Amino-2-methoxyquinoline was reacted with 4-(4-pyridyloxy)aniline according to Method B2c to afford the urea.

Entry 26: 3-Amino-2-methoxyquinoline was reacted with 4-((4-methoxyphenyl)methylamino)aniline according to Method B4 to afford the urea.

Entry 27: 3-(4-Pyridylthio)aniline was prepared according to Method A4a. 3-Amino-2-methoxyquinoline and 3-(4-pyridylmethyl)aniline were reacted according to Method B3 to afford the urea.

Entry 28: 4-(4-Pyridyloxy)aniline was prepared starting from 4-hydroxypyridine and 1-fluoro-4-nitrobenzene according to Method A3d. 1-(4-Methylpiperazinyl)-3-aminoisoquinoline was reacted with 4-(4-aminophenoxy)pyridine according to Method B2a to afford the urea.

Entry 104: 4-(4-(2-(*N*-Methylcarbamoyl)pyridyloxy)aniline was prepared according to Method A10. 3-Amino-2-methoxyquinofine was reacted with 4-(4-(2-(*N-*methylcarbamoyl)pyridyloxy)aniline according to Method B4 to afford the urea.

Entry 105: 4-(3-*N*-Methylcarbamoylphenoxy)aniline was prepared according to Method A14. 3-Amino-2-methoxyquinoline was reacted with 4-(3-*N-*methylcarbamoylphenoxy)aniline according to Method B4 to afford the urea.

Entry 106: 4-Chloropyridine-2-carbonyl chloride was reacted with isopropylamine according to Method A10, Step 3b. The resulting 4-chloro-*N*-isopropyl-2-pyridinecarboxamide was reacted with 4-aminophenol according to Method A10, Step 4 to give 4-(2-(*N*-isopropylcarbamoyl)-4-pyridyloxy)aniline. 3-Amino-2-methoxyquinoline was reacted with 4-(2-(*N*-isopropylcarbamoyl)-4-pyridyloxy)aniline according to Method B5b to afford the urea.

Entry107: 4-Chloropyridine-2-carbonyl chloride HCl salt was reacted with ammonia according to Method A10, Step 3b to form 4-chloro-2-pyridinecarboxamide. 4-Chloro-2-pyridinecarboxamide was reacted with 4-aminophenol according to Method A10, Step 4 using DMAC in place of DMF to give 4-(2-carbamoyl-4-pyridyloxy)aniline. 4-(2-Carbamoyl-4-pyridyloxy)aniline was reacted with 4-(2-(*N*-isopropylcarbamoyl)-4-pyridyloxy)aniline according to Method B6 to afford the urea.

Entry108: 4-Chloro-*N*-methyl-2-pyridinecarboxamide was synthesized according to Method A10, Step 3b. 4-Chloro-*N*-methyl-2-pyridinecarboxamide was reacted with 4-aminophenol according to Method A10, Step 4 using DMAC in place of DMF to give 4-(2-(*N*-methylcarbamoyl)-4-pyridyloxy)aniline. 3-Amino-2-methoxyquinoline was reacted with 4-(2-(*N*-methylcarbamoyl)-4-pyridyloxy)aniline according to Method B6 to afford the urea.

Entry109: 4-Chloropyridine-2-carbonyl chloride HCl salt was reacted with ammonia according to Method A10, Step 3b to form 4-chloro-2-pyridinecarboxamide. 4-Chloro-2-pyridinecarboxamide was reacted with 3-aminophenol according to Method A10, Step 4 using DMAC in place of DMF to give 3-(2-carbamoyl-4-pyridyloxy)aniline. 3-Amino-2-methoxyquinoline was reacted with 3-(2-carbamoyl-4-pyridyloxy)aniline according to Method B6 to afford the urea.

Entry110: 4-Chloro-*N*-methyl-2-pyridinecarboxamide, which was synthesized according to Method A10, Step 3a, was reacted with 3-aminophenol according to Method A10, Step 4 using DMAC in place of DMF to give 3-(-2-(*N*-methylcarbamoyl)-4-pyridyloxy)aniline. 3-Amino-2-methoxyquinoline was reacted with 3-(-2-(*N-*methylcarbamoyl)-4-pyridyloxy)aniline according to Method B6 to afford the urea.

Entry 111: 4-(4-(3-(*N*-Methylcarbamoyl)-2-methoxyphenoxy)aniline was prepared according to Method A13. 3-Amino-2-methoxyquinoline was reacted with 4-(4-(3-(*N-*Methylcarbamoyl)-2-methoxyphenoxy)aniline was according to Method B6 to afford the urea.

Entry 112: 5-(4-Aminophenoxy)isoindoline-1,3-dione was prepared according to Method A11. 3-Amino-2-methoxyquinoline was reacted with 5-(4-Aminophenoxy)isoindoline-1,3-dione was according to Method B5b to afford the urea.

The following compounds have been synthesized according to the General Methods listed above:

**Table 1. 4-tert-Butyl-2-pyridyl Ureas**

| | | | | | | |
|---|---|---|---|---|---|---|
| 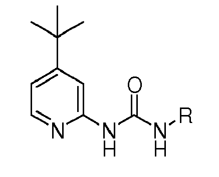 | | | | | | |

| Entry | **R** | mp (°C) | HPLC (min.) | TLC R*_{f}* | TLC Solvent System | Mass Spec. [Source] |
|---|---|---|---|---|---|---|
| 1 | 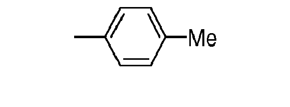 | | | 0.51 | 33% EtOAc / 67% hexane | 284 (M+H)+ (FAB) |
| 2 | 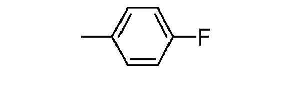 | | | 0.45 | 33% EtOAc / 67% hexane | 288 (M+H)+ (FAB) |
| 3 | 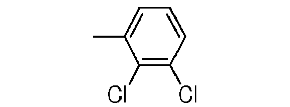 | | | 0.40 | 33% EtOAc / 67% hexane | 338 (M+H)+ (FAB) |
| 4 | 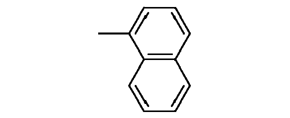 | | | 0.46 | 33% EtOAc / 67% hexane | 320 (M+H)+ (FAB) |
| 5 | 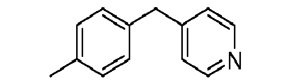 | | | 0.47 | 100% EtOAc | 361 (M+H)+ (FAB) |
| 6 | 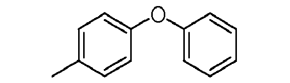 | 179 -180 | | 0.58 | 5% MeOH /95% CH2Cl2 | 362 (M+H)+ (FAB) |
| 7 | 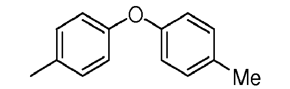 | 190 -191 | | 0.46 | 5% MeOH /95% CH2Cl2 | 376 (M+H)+ (FAB) |
| 8 | 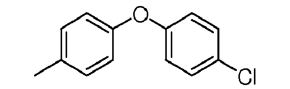 | 198 -199 | | 0.76 | 5% MeOH /95% CH2Cl2 | 396 (M+H)+ (FAB) |
| 9 | 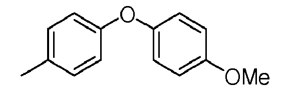 | 189 -193 | | 0.43 | 5% MeOH /95% CH2Cl2 | 392 (M+H)+ (FAB) |
| 10 | 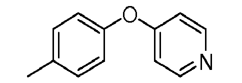 | | | 0.40 | 100% EtOAc | 363 (M+H)+ (FAB) |
| 11 | 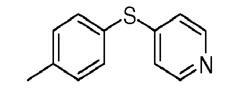 | 208 -212 | | 0.39 | 5% MeOH /95% CH2Cl2 | 379 (M+H)+ (HPLC ES-MS) |
| 12 | 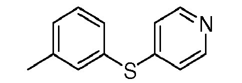 | 196-197 | | 0.37 | 5% MeOH /95% CH2Cl2 | 379 (M+H)+ (FAB) |

**Table 2. 5-(Trifluoromethyl)-2-pyridyl Ureas**

| | | | | | | |
|---|---|---|---|---|---|---|
| 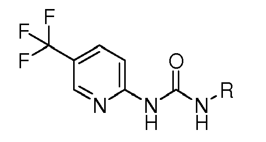 | | | | | | |

| Entry | **R** | mp (°C) | HPLC (min.) | TLC R*_{f}* | TLC Solvent System | Mass Spec. [Source] |
|---|---|---|---|---|---|---|
| 13 | 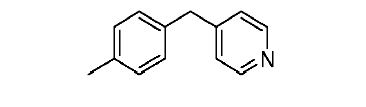 | | | 0.34 | 30% acetone / 70% CH2Cl2 | 373 (M+H)+ (FAB) |
| 14 | 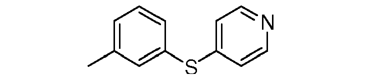 | | | 0.33 | 50% EtOAc /50% hexane | 391 (M+H)+ (FAB) |

**Table 3. 3-Isoquinolyl Ureas**

| | | | | | | |
|---|---|---|---|---|---|---|
| 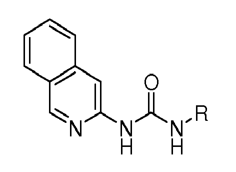 | | | | | | |

| Entry | **R** | mp (°C) | HPLC (min.) | TLC R*_{f}* | TLC Solvent System | Mass Spec. [Source] |
|---|---|---|---|---|---|---|
| 15 | 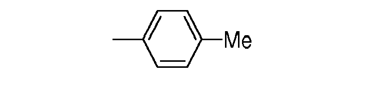 | | | 0.60 | 50% EtOAc / 50% hexane | 278 (M+H)+ (FAB) |
| 16 | 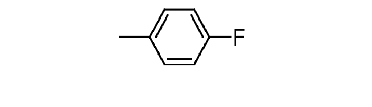 | | | 0.52 | 50% EtOAc / 50% hexane | 282 (M+H)+ (FAB) |
| 17 | 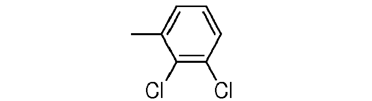 | | | 0.75 | 50% EtOAc / 50% hexane | 322 (M+H)+ (FAB) |
| 18 | 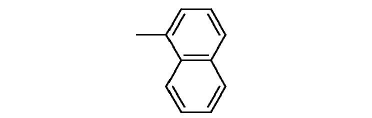 | | | 0.57 | 50% EtOAc / 50% hexane | 314 (M+H)+ (FAB) |
| 19 | 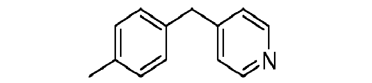 | | | 0.35 | 100% EtOAc | 355 (M+H)+ (FAB) |
| 20 | 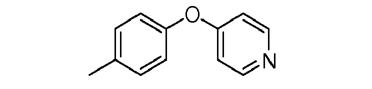 | | | 0.27 | 100% EtOAc | 357 (M+H)+ (FAB) |

**Table 4. 3-Quinolyl Ureas**

| | | | | | | |
|---|---|---|---|---|---|---|
| 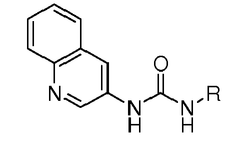 | | | | | | |

| Entry | **R** | mp (°C) | HPLC (min.) | TLC R*_{f}* | TLC Solvent System | Mass Spec. [Source] |
|---|---|---|---|---|---|---|
| 21 | 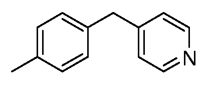 | | | 0.25 | 60% acetone / 40% CH2Cl2 | 355 (M+H)+ (FAB) |

**Table 5. 2-Methoxy-3-quinolyl Ureas**

| | | | | | | |
|---|---|---|---|---|---|---|
| 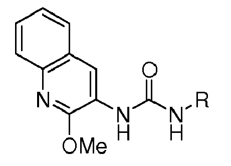 | | | | | | |

| Entry | **R** | mp (°C) | HPLC (min.) | TLC *R_{f}* | TLC Solvent System | Mass Spec. [Source] |
|---|---|---|---|---|---|---|
| 22 | 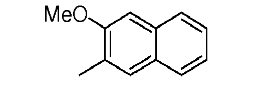 | | | 0.45 | 50% EtOAc / 50% hexane | 375 (M+H)+ (HPLC ES-MS) |
| 23 | 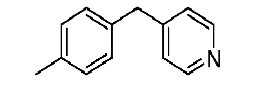 | | | 0.56 | 50% EtOAc / 50% hexane | 385 (M+H)+ (FAB) |
| 24 | 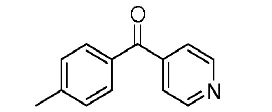 | | | 0.45 | 100% EtOAc | 399 (M+H)+ (FAB) |
| 25 | 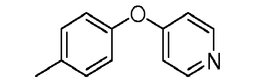 | 207 -208 | | 0.24 | 5% MeOH /95% CH2Cl2 | 387 (M+H)+ (FAB) |
| 26 | 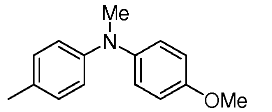 | 126 -130 | | | | |
| 27 | 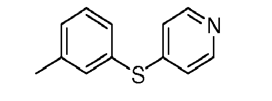 | | | 0.39 | 50% acetone / 50% CH2Cl2 | 403 (M+H)+ (FAB) |

**Table 6. 3-Quinolyl Ureas**

| | | | | | | |
|---|---|---|---|---|---|---|
| 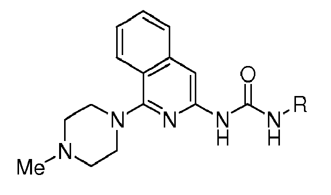 | | | | | | |

| Entry | **R** | mp (°C) | HPLC (min.) | TLC R*_{f}* | TLC Solvent System | Mass Spec. [Source] |
|---|---|---|---|---|---|---|
| 28 | 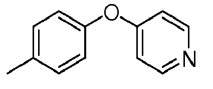 | | | 0.20 | 30% MeOH / 70% EtOAc | 455 (M+H)+ (HPLC ES-MS) |

**Table 7. Additional Isoquinolyl Ureas**

| | | | | | | | |
|---|---|---|---|---|---|---|---|
| 104 | 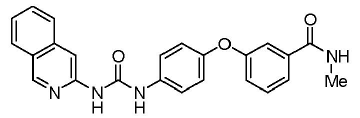 | | | 0.30 | 1% Et3N/ 99% EtOAc | 414 (M+H)+ (HPLC ES-MS) | A2 C5 |

**Table 8. 2-Methoxy-3-quinolyl Ureas with Omega Carbonyls**

| | | | | | | | |
|---|---|---|---|---|---|---|---|
| 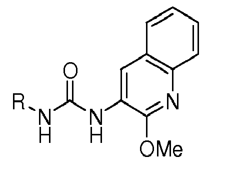 | | | | | | | |

| Entry | **R** | mp (°C) | HPLC (min.) | TLC R*_{f}* | TLC Solvent System | Mass Spec. [Source] | Synth. Method |
|---|---|---|---|---|---|---|---|
| 105 | 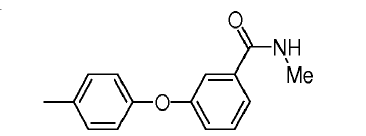 | 213-214 | | 0.20 | 5% MeOH/ 95% CHCl3 | 443 (M+H)+ (FAB) | A13 C2c |
| 106 | 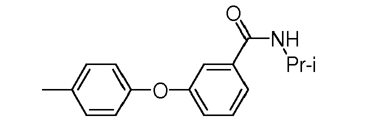 | 244-245 | | | | | A2 C2c |
| 107 | 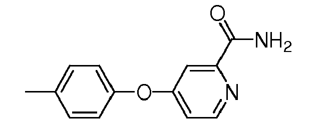 | | | 0.52 | 100% EtOAc | 430 (M+H)+ (FAB) | A2 C5 |
| 108 | 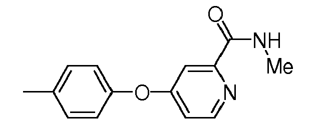 | | | 0.55 | 100% EtOAc | 444 (M+H)+ (FAB) | A2 C5 |
| 109 | 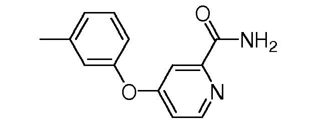 | | | 0.30 | 100% EtOAc | 430 (M+H)+ (FAB) | A2 C5 |
| 110 | 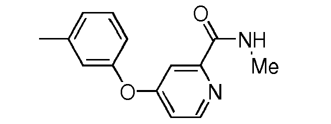 | | | 0.60 | 100% EtOAc | 444 (M+H)+ (FAB) | A2 C5 |
| 111 | 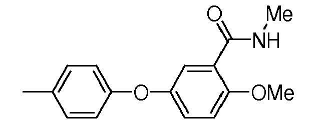 | 144-146 | | | | | A8 C5 |
| 112 | 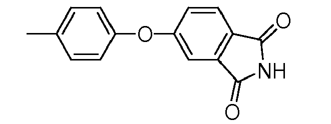 | | | | | | A3 C2c |

### BIOLOGICAL EXAMPLES

### P38 Kinase Assay:

The *in vitro* inhibitory properties of compounds were determined using a p38 kinase inhibition assay. P38 activity was detected using an *in vitro* kinase assay run in 96-well microtiter plates. Recombinant human p38 (0.5 µg/mL) was mixed with substrate (myelin basic protein, 5 µg/mL) in kinase buffer (25 mM Hepes, 20 mM MgCl₂ and 150 mM NaCl) and compound. One µCi/well of ³³P-labeled ATP (10 µM) was added to a final volume of 100 µL. The reaction was run at 32 °C for 30 min. and stopped with a 1M HCl solution. The amount of radioactivity incorporated into the substrate was determined by trapping the labeled substrate onto negatively charged glass fiber filter paper using a 1% phosphoric acid solution and read with a scintillation counter. Negative controls include substrate plus ATP alone.

All compounds exemplified displayed p38 IC₅₀s of between 1 nM and 10 µM.

### LPS Induced TNFα Production in Mice:

The *in vivo* inhibitory properties of selected compounds were determined using a murine LPS induced TNFα production *in vivo* model. BALB/c mice (Charles River Breeding Laboratories; Kingston, NY) in groups of ten were treated with either vehicle or compound by the route noted. After one hour, endotoxin (E. coli lipopolysaccharide (LPS) 100 µg) was administered intraperitoneally (i.p.). After 90 min, animals were euthanized by carbon dioxide asphyxiation and plasma was obtained from individual animals by cardiac puncture into heparinized tubes. The samples were clarified by centrifugation at 12,500 x g for 5 min at 4 °C. The supernatants were decanted to new tubes, which were stored as needed at -20 °C. TNFα levels in sera were measured using a commercial murine TNF ELISA kit (Genzyme).

The preceding examples can be repeated with similar success by substituting the generically or specifically described reactants and/or operating conditions of this invention for those used in the preceding examples.

From the foregoing discussion, one skilled in the art can easily ascertain the essential characteristics of this invention and, without departing from the spirit and scope thereof, can make various changes and modifications of the invention to adapt it to various usages and conditions.

## Claims

1. A compound of the following formula
A'- D - B' (I)
or a pharmaceutically acceptable salt thereof, wherein
D is -NH-C(O)-NH-,
A' is selected from the group consisting of substituted t-butylpyridinyl, unsubstituted t-butylpyridinyl, substituted (trifluoromethyl)pyridyl, unsubstituted (trifluoromethyl)pyridyl,
B' is a substituted aryl ring having 6 cyclic members and is substituted by -CN, halogen, C₁₋₁₀ alkyl, C₁₋₁₀ alkoxy, -OH, up to per halo substituted C₁₋₁₀ alkyl, up to per halo substituted C₁₋₁₀ alkoxy, -OR⁷, -SR⁷, -NR⁷R^{7'}, -NR⁷C(O)OR^{7'}, -NR⁷C(O)R^{7'}, or -NO₂, wherein each R⁷ and R^{7'} is independently selected from hydrogen, C₁₋₁₀ alkyl, C₁₋₁₀ alkoxy, C₂₋₁₀ alkenyl, C₁₋₁₀ alkenoyl, up to per halosubstituted C₁₋₁₀ alkyl, up to per halosubstituted C₁₋₁₀ alkoxy, up to per halosubstituted C₂₋₁₀ alkenyl and up to per halosubstituted C₁₋₁₀ alkenoyl.

2. A compound of claim 1, wherein A' has 1-3 substituents selected from the group consisting of C₁₋₁₀ alkyl, up to per halo substituted C₁₋₁₀ alkyl, -CN, -OH, halogen, C₁₋₁₀ alkoxy, up to per halo substituted C₁₋₁₀ alkoxy and C₃₋₁₀ heterocyclic moieties having at least five cyclic members and 1 to 2 heteroatoms selected from the group of consisting of nitrogen, oxygen and sulfur.

3. A compound of either of claims 1 or 2 which is a pharmaceutically acceptable salt of a compound of formula I' selected from the group consisting of
a) basic salts of organic acids and inorganic acids selected from the group consisting of hydrochloric acid, hydrobromic acid, sulfuric acid, phosphoric acid, methanesulfonic acid, trifluorosulfonic acid, benzenesulfonic acid, p-toluene sulfonic acid (tosylate salt), 1-napthalene sulfonic acid, 2-napthalene sulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid; and
b) acid salts of organic and inorganic bases containing cations selected from the group consisting of alkaline cations, alkaline earth cations, the ammonium cation, aliphatic substituted ammonium cations and aromatic substituted ammonium cations.

4. A compound selected from the group consisting of
N-(4-tert-butylpyridinyl)-N'(4-methylphenyl) urea
N-(4-tert-butylpyridinyl)-N'-(4-fluorophenyl) urea
N-(4-tert-butylpyridinyl)-N'-(2,3-dichlorophenyl) urea
N-(4-tert-butylpyridinyl)-N'-(1-naphthyl) urea
N-(4-tert-butylpyridinyl)-N'-(4-)4-methoxyphenoxy)phenyl) urea
N-(2-)(5-trifluoromethyl)pyridinloxy-N'-(4-)4-pyridylmethyl)phenyl) urea
N-(2-)(5-trifluoromethyl)pyridinloxy-N'-(3-)4-pyridylthio)phenyl) urea
N-(3-isoquinolyl)-N'-(4-methylphenyl) urea
N-(3-isoquinolyl)-N'-(4-fluorophenyl) urea
N-(3-isoquinolyl)-N'-(2,3-dichlorophenyl) urea
N-(3-isoquinolyl)-N'-(1-naphthyl) urea
N-(3-isoquinolyl)-N'-(4-)4-pyridinylmethyl)phenyl) urea
N-(3-quinolyl)-N'-(4-)4-pyridinylmethyl)phenyl) urea.

5. A pharmaceutical composition comprising a compound of any one of claims 1 to 4 and a physiologically acceptable carrier.

6. A compound of any one of claims 1 to 4 for use in treating a disease mediated by p38 within a host.

7. A compound for use in treating a disease mediated by p38 within a host according to claim 6, wherein the disease mediated by p38 within a host is rheumatoid arthritis, osteoarthritis, septic arthritis, tumor metastasis, periodontal disease, corneal ulceration, proteinuria, coronary thrombosis from atherosclerotic plaque, aneurysmal aortic, birth control, dystrophobic epidermolysis bullosa, degenerative cartilage loss following traumatic joint injury, osteopenias mediated by MMP activity, tempero mandibular joint disease, demyelating disease of the nervous system, rheumatic fever, bone resorption, postmenopausal osteoperosis, sepsis, gram negative sepsis, septic shock, endotoxic shock, toxic shock syndrome, systemic inflammatory response syndrome, inflammatory bowel disease (Crohn's disease and ulcerative colitis), Jarisch-Herxheimer reaction, asthma, adult respiratory distress syndrome, acute pulmonary fibrotic disease, pulmonary sarcoidosis, allergic respiratory disease, silicosis, coal worker's pneumoconiosis, alveolar injury, hepatic failure, liver disease during acute inflammation, severe alcoholic hepatitis, malaria (Plasmodium falciparum malaria and cerebral malaria), non-insulin-dependent diabetes mellitus (NIDDM), congestive heart failure, damage following heart disease, atherosclerosis, Alzheimer's disease, acute encephalitis, brain injury, multiple sclerosis (demyelation and oligiodendrocyte loss in multiple sclerosis), advanced cancer, lymphoid malignancy, pancreatitis, impaired wound healing in infection, inflammation and cancer, myelodysplastic syndromes, systemic lupus erythematosus, biliary cirrhosis, bowel necrosis, psoriasis, radiation injury/toxicity following administration of monoclonal antibodies, host-versus-graft reaction (ischemia reperfusion injury and allograft rejections of kidney, liver, heart, and skin), lung allograft rejection (obliterative bronchitis), complications due to total hip replacement, tuberculosis, Helicobacter pylori infection during peptic ulcer disease, Chaga's disease resulting from Trypanosoma cruzi infection, effects of Shiga-like toxin resulting from E. coli infection, effects of enterotoxin A resulting from Staphylococcus infection, meningococcal infection, and infections from Borrelia burgdorferi, Treponema pallidum, cytomegalovirus, influenza virus, Theiler's encephalomyelitis virus or the human immunodeficiency virus (HIV).
